# EUROPEAN PATENT APPLICATION

(11) **EP 3 315 596 A1**
(43) Date of publication of application: **02.05.2018**
(21) Application number: 16814544.9
(22) Date of filing: 24.06.2016
(51) Int. Cl.: C12M 3/00, C12M 1/22

(54) **CELL CULTURE CONTAINER**

(30) Priority: 26.06.2015 JP 2015129266; 15.10.2015 JP 2015203835
(71) Applicant: National Cerebral and Cardiovascular Center, Suita-shi, Osaka 565-8565 (JP)
(72) Inventor: NAKAYAMA, Yasuhide, Suita-shi Osaka 565-8565 (JP); IWAI, Ryosuke, Suita-shi Osaka 565-8565 (JP); NEMOTO, Yasushi, Tokyo 104-8340 (JP)
(74) Representative: Carpmaels & Ransford LLP
(86) International application number: PCT/JP2016/069565
(87) International publication number: WO 2016/208777

(57) **Abstract**

The present disclosure aims to provide a cell culture container capable of easily producing large quantities of minute cellular structures. A cell culture container includes a plurality of first coated regions, on a culture surface of the cell culture container, coated by a temperature-responsive polymer or a temperature-responsive polymer composition. The surface zeta potential of the first coated regions is from 0 mV to 50 mV.

## Description

### TECHNICAL FIELD

The present disclosure relates in particular to a cell culture container capable of easily producing large quantities of minute cellular structures.

### BACKGROUND

Important experimental techniques in the field of biology include cell culture techniques developed around the year 1900. Initial development of these techniques focused only on conditioning cells, such as optimizing the medium components, and techniques for monolayer cultures and suspension cultures were mainly studied.

In recent years, it has become clear that various properties, stimulus responsiveness, cell functions, and the like differ between monolayer cultured cells and cells in living tissue. Instead of monolayer cultures that form a monolayer structure, demand is increasing for 3D cultures, in particular spheroid cultures, that cause a 3D structure resembling the tissue structure in a living organism to form.

Traditional techniques that have been actively developed include a technique for embedding cells in a 3D gel formed by floating cells in a protein solution and causing the cell suspension to gel in reaction to a certain trigger (heat, light, a chemical crosslinking agent, or the like), a technique for grafting cells onto a porous scaffold, and a technique for manufacturing a laminate of a cell sheet using a culture dish with hardened NIPAM.

In recent years, methods such as the following have been developed: a method for precipitating cells to a non-adhesive round bottom of Prime Surface, by Sumitomo Bakelite Co., Ltd., or the like; a method for heightening the migration property of cells adhered to a culture surface by providing a smooth surface, such as a Nano Culture Plate by JSR Co. or a Nano Pillar Plate by Hitachi Ltd., with a regular pattern of unevenness by laser processing, thereby inducing self-assembly of cells on the culture surface; and a method for using a culture dish with numerous holes approximately 100 µ to 500 µ in diameter and 500 µm deep, such as Elplasia by Kuraray Co., Ltd. or EZSPHERE by Iwaki & Co., Ltd., to precipitate cells seeded in each hole to the bottom of the hole.

### CITATION LIST

### Non-patent Literature

NPL 1: Nature, Vol 424, P870-872, 21 August, 2003.

### SUMMARY

### (Technical Problem)

However, in the case of producing large quantities of cellular structures, such as spheroids, that have a 3D structure, cells need to be seeded in numerous minute wells (for example, 100 µm × 200 µm) in the above-described known cell culture container, making it difficult to manipulate the culture.

It would therefore be helpful to provide a cell culture container capable of easily producing large quantities of minute (for example, with a size of several hundred µm or less) cellular structures (for example, spheroids).

### (Solution to Problem)

A cell culture container of the present disclosure includes a plurality of first coated regions, on a culture surface of the cell culture container, coated by a temperature-responsive polymer or a temperature-responsive polymer composition, wherein a surface zeta potential of the first coated regions is from 0 mV to 50 mV.

In the cell culture container of the present disclosure, a contact angle of water relative to the first coated regions is preferably from 50° to 90°.

In the cell culture container of the present disclosure, the culture surface of the cell culture container is preferably cell non-adhesive.

In the cell culture container of the present disclosure, a shape of the first coated regions in planar view is preferably at least one selected from the group consisting of a circle, an ellipse, and a shape with a center of curvature on an inside of an outer contour line.

In the cell culture container of the present disclosure, the temperature-responsive polymer or temperature-responsive polymer composition is preferably at least one temperature-responsive polymer or temperature-responsive polymer composition selected from the group consisting of a temperature-responsive polymer containing 2-N,N-dimethylaminoethyl methacrylate (DMAEMA) units and anionic monomer units; a temperature-responsive polymer containing N-isopropyl acrylamide (NIPAM) units, cationic monomer units, and anionic monomer units; and a temperature-responsive polymer composition containing a polymer of 2-N,N-dimethylaminoethyl methacrylate (DMAEMA) and/or a derivative thereof, 2-amino-2-hydroxymethyl-1,3-propanediol (tris), and one or more anionic substances selected from the group consisting of nucleic acids, heparin, hyaluronic acid, dextran sulfate, polystyrene sulfonic acid, polyacrylic acid, polymethacrylic acid, polyphosphoric acid, sulfated polysaccharide, curdlan, polyarginic acid, and alkali metal salts thereof.

In the cell culture container of the present disclosure, the anionic monomer is preferably at least one selected from the group consisting of acrylic acid, methacrylic acid, vinyl derivatives containing at least one group selected from the group consisting of a carboxyl group, a sulfonic acid group, and a phosphoric acid group in a side chain.

In the cell culture container of the present disclosure, the cationic monomer is preferably at least one selected from the group consisting of 3-(N,N-dimethylaminopropyl)-(meth)acrylamide, 3-(N,N-dimethylaminopropyl)-(meth)acrylate, aminostyrene, 2-(N,N-dimethylaminoethyl)-(meth)acrylamide, and 2-(N,N-dimethylaminoethyl)-(meth)acrylate.

The cell culture container of the present disclosure may further include second coated regions further coated with a cell adhesive material, the second coated regions being located inside the first coated regions.

In the cell culture container of the present disclosure, the cell adhesive material is preferably at least one selected from the group consisting of laminin, collagen, and fibronectin.

### (Advantageous Effect)

The present disclosure can provide a cell culture container capable of easily producing large quantities of minute (for example, with a size of several hundred µm or less) cellular structures (for example, spheroids).

### BRIEF DESCRIPTION OF THE DRAWINGS

In the accompanying drawings:

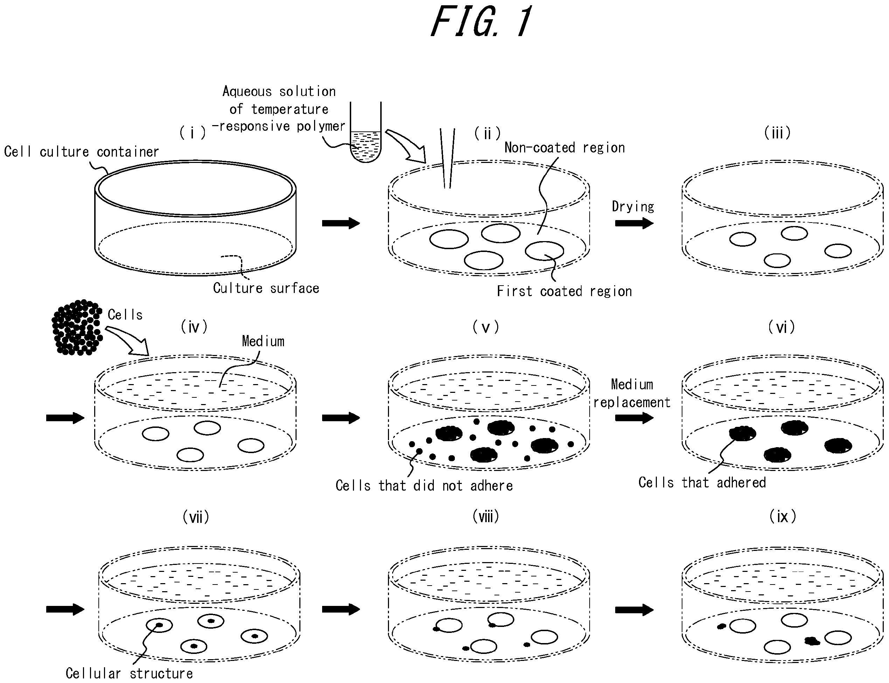

FIG. 1 illustrates an outline of an example of a cell culture container according to an embodiment of the present disclosure, along with an outline of an example of a manufacturing method of a cell culture container according to an embodiment of the present disclosure and an outline of an example of a cell culture method using a cell culture container according to an embodiment of the present disclosure;

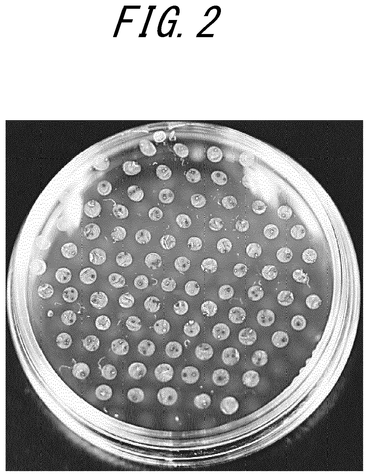

FIG. 2 is a view from the culture surface of the cell culture container of an example of the present disclosure produced in Test 4;

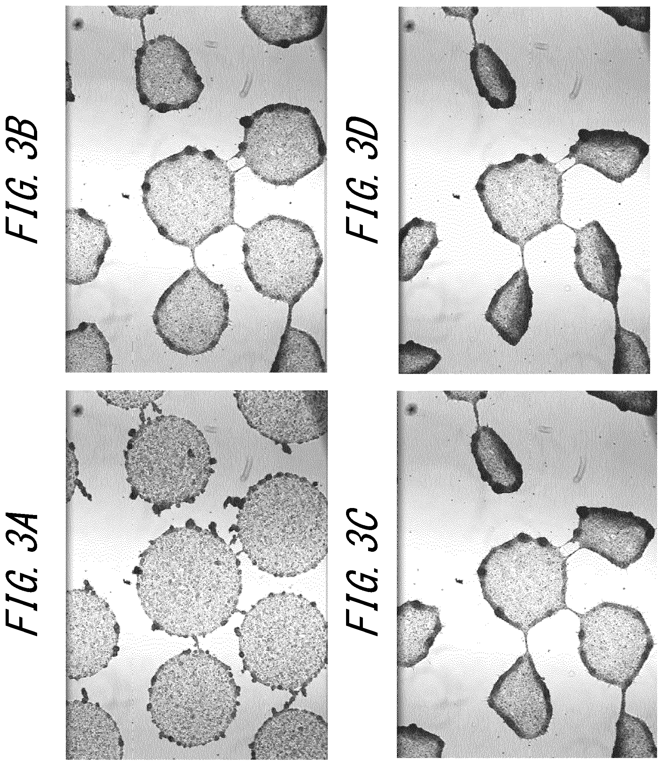

FIGS. 3A to 3D are photographs taken using a phase contrast microscope to observe the state when culturing adipose stem cells derived from rat subcutaneous fat in first coated regions of a cell culture container of an example of the present disclosure, respectively illustrating the state at zero hours (immediately after medium replacement), 2 hours, 3 hours, and 4 hours after medium replacement;

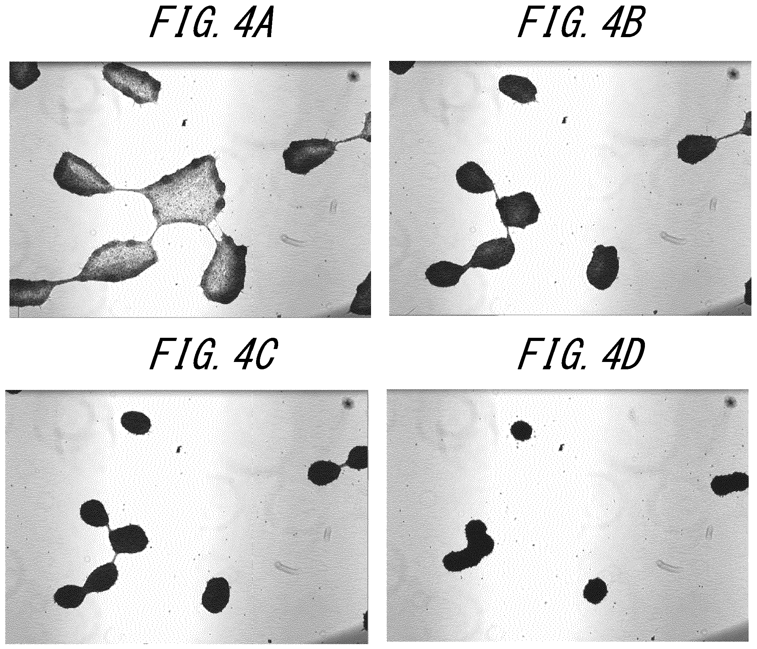

FIGS. 4A to 4D are photographs taken using a phase contrast microscope to observe the state when culturing adipose stem cells derived from rat subcutaneous fat in first coated regions of a cell culture container of an example of the present disclosure, respectively illustrating the state at 5 hours, 6 hours, 8 hours, and 12 hours after medium replacement;

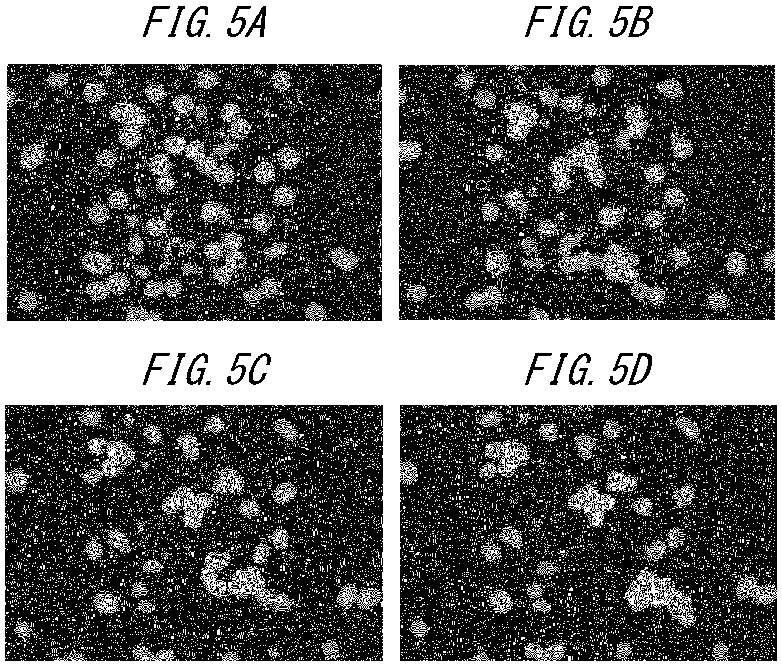

FIGS. 5A to 5D are photographs taken using a fluorescence microscope to observe the state when culturing adipose stem cells derived from rat subcutaneous fat in first coated regions of a cell culture container of an example of the present disclosure, respectively illustrating the state at zero hours, 5 hours, 8 hours, and 12 hours after aggregated cellular structures detach from the first coated regions spontaneously and float, and the cell culture container is appropriately tipped and mixed so that the suspended matter is artificially collected in the center of the cell culture container and the cellular structures come into contact with each other;

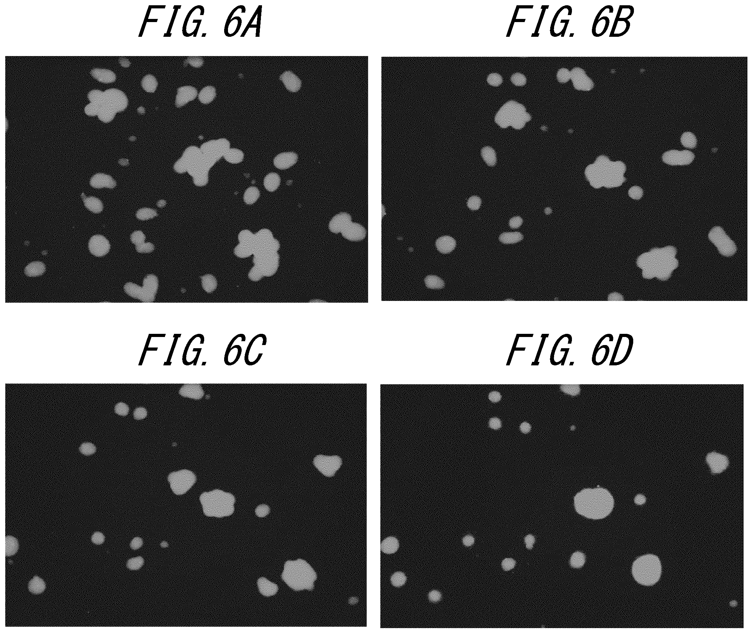

FIGS. 6A to 6D are photographs taken using a fluorescence microscope to observe the state when culturing adipose stem cells derived from rat subcutaneous fat in first coated regions of a cell culture container of an example of the present disclosure, respectively illustrating the state at 17 hours, 22 hours, 25 hours, and 34 hours after aggregated cellular structures detach from the first coated regions spontaneously and float, and the cell culture container is appropriately tipped and mixed so that the suspended matter is artificially collected in the center of the cell culture container and the cellular structures come into contact with each other;

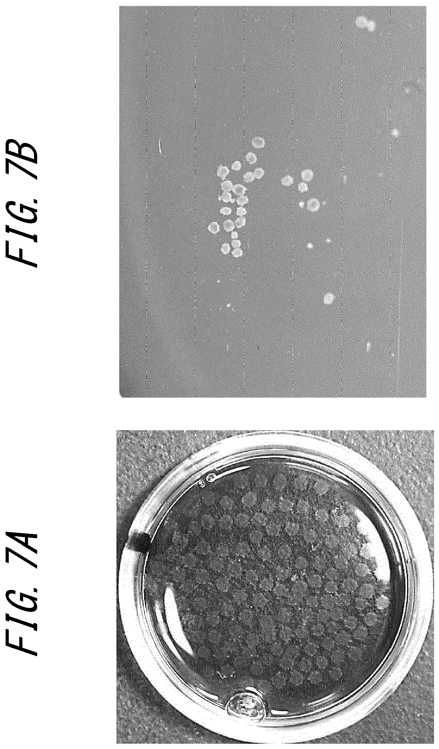

FIG. 7A is a photograph taken using a stereomicroscope to observe the state zero hours after medium replacement (immediately after medium replacement) when culturing adipose stem cells derived from rat subcutaneous fat in first coated regions of a cell culture container of an example of the present disclosure, and FIG. 7B is a photograph taken using a stereomicroscope to observe the state 21 hours after medium replacement when culturing adipose stem cells derived from rat subcutaneous fat in first coated regions of a cell culture container of an example of the present disclosure;

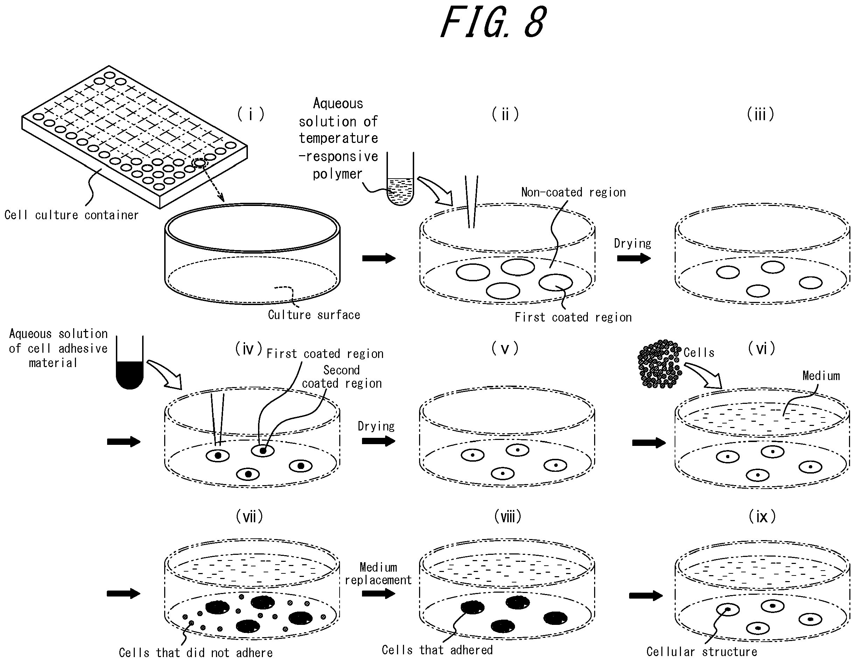

FIG. 8 illustrates an outline of another example of a cell culture container according to an embodiment of the present disclosure, along with an outline of another example of a manufacturing method of a cell culture container according to an embodiment of the present disclosure and an outline of another example of a cell culture method using a cell culture container according to an embodiment of the present disclosure;

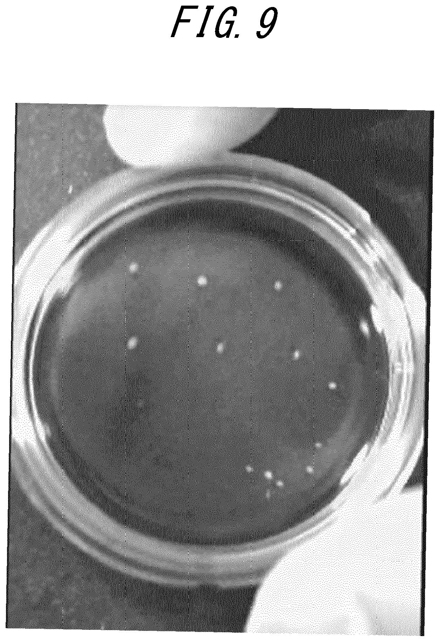

FIG. 9 is a photograph taken using a stereomicroscope to observe the state zero hours after medium replacement (immediately after medium replacement) when culturing adipose stem cells derived from rat subcutaneous fat in first and second coated regions of a cell culture container of an example of the present disclosure;

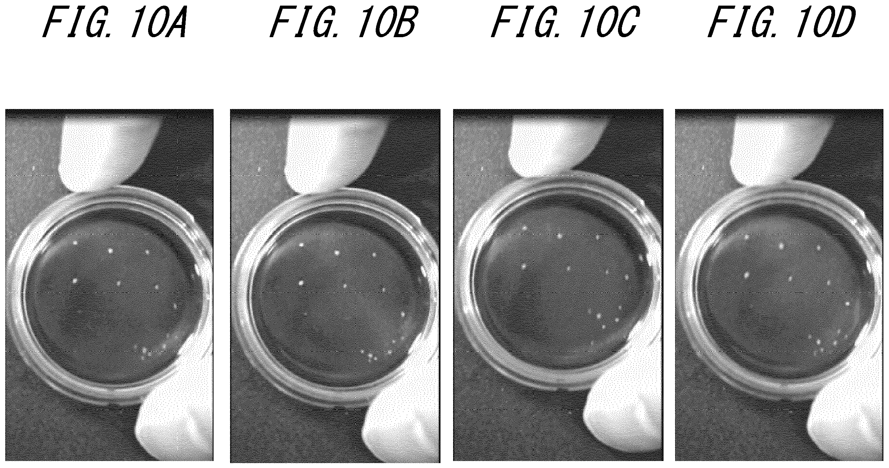

FIGS. 10A to 10D are photographs taken using a phase contrast microscope to observe the state when the cellular structure illustrated in FIG. 9 was shaken by hand in all four directions in a horizontal plane at a speed of approximately 10 cm/s, respectively illustrating the state at 0 s, 0.5 s, 1.0 s, and 1.5 s after the start of shaking;

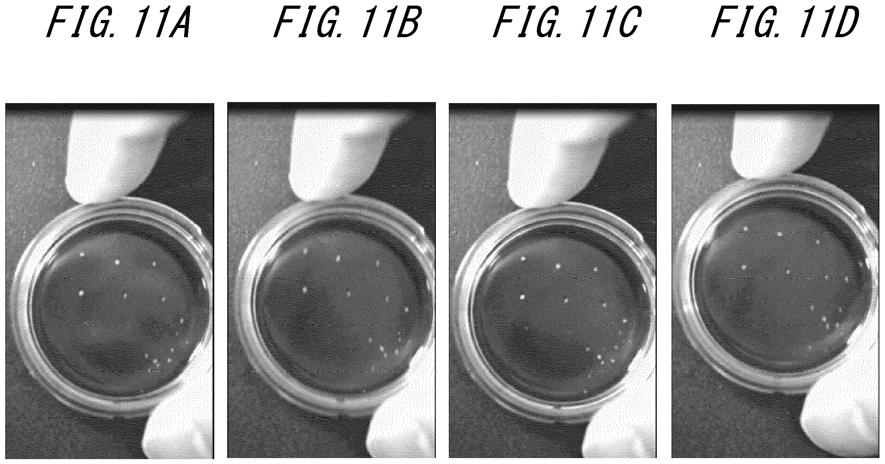

FIGS. 11A to 11D are photographs taken using a phase contrast microscope to observe the state when the cellular structure illustrated in FIG. 9 was shaken by hand in all four directions in a horizontal plane at a speed of approximately 10 cm/s, respectively illustrating the state at 2.0 s, 2.5 s, 3.0 s, and 3.5 s after the start of shaking; and

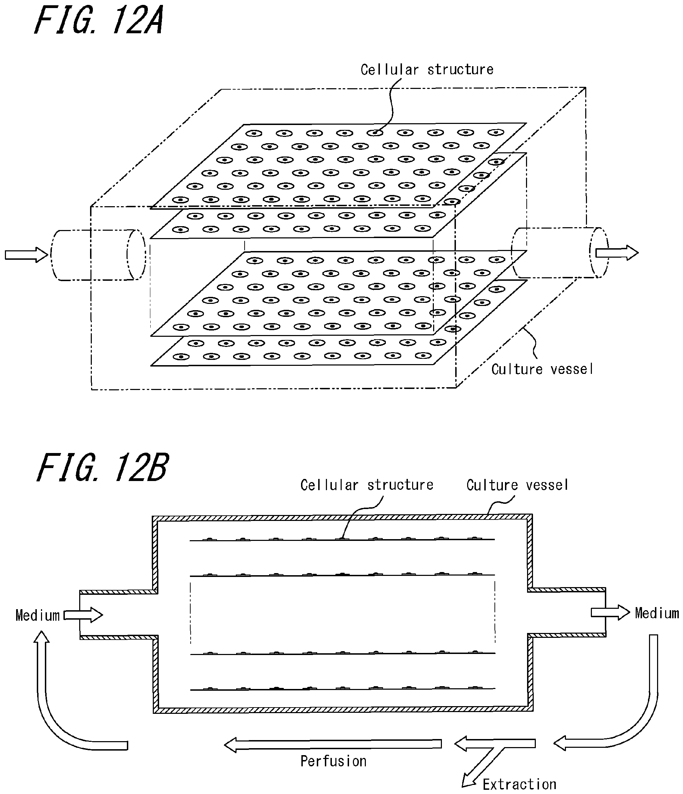

FIGS. 12A and 12B illustrate an outline of a cellular structure culture vessel according to an embodiment of the present disclosure using another example of a cell culture container according to an embodiment of the present disclosure, where FIG. 12A is a perspective view of the cellular structure culture vessel, and FIG. 12B is a cross-sectional view of the cellular structure culture vessel.

### DETAILED DESCRIPTION

An embodiment of a cell culture container according to the present disclosure is described below in detail with examples.

FIG. 1 illustrates an outline of a cell culture container according to an embodiment of the present disclosure, along with an outline of an example of a manufacturing method of a cell culture container according to an embodiment of the present disclosure and an outline of an example of a cell culture method using a cell culture container according to an embodiment of the present disclosure.

A cell culture container according to an embodiment of the present disclosure (cell culture container of the present embodiment) includes a plurality of first coated regions, on the culture surface of the cell culture container, coated by a temperature-responsive polymer or a temperature-responsive polymer composition.

Examples of the cell culture container include commercially available plates, dishes, flasks, glass plates, silicone sheets, and the like.

The surface zeta potential of the first coated regions coated by a temperature-responsive polymer or a temperature-responsive polymer composition in the cell culture container of the present embodiment is from 0 mV to 50 mV. Setting the surface zeta potential to 0 mV or greater allows adhesion of negatively charged cells, while setting the surface zeta potential to 50 mV or less reduces cytotoxicity.

For similar reasons, the surface zeta potential is preferably from 0 mV to 35 mV and more preferably from 10 mV to 25 mV.

After using this cell culture container to culture cells in a typical 37°C incubator and form tissue, cells with a cell sheet structure, for example, can be collected without performing a cell detachment operation such as trypsinization.

Furthermore, a cell culture container according to an embodiment of the present disclosure that has a surface zeta potential in the aforementioned particular range allows a cellular structure (spheroid) having an aggregated (pellet-like) structure to be formed easily by simply culturing cells under appropriate culture conditions.

The reason is that setting the surface zeta potential within the aforementioned particular range is inferred to provide the culture surface of the cell culture container with a weak positive charge that does not trigger cytotoxicity, ensure rapid adhesion of the seeded cells, improve the activity of cells and encourage secretion of the extracellular matrix, and also appropriately inhibit cell migration, strengthening the bond between cells.

The phenomenon whereby the aforementioned cellular structure is formed is extremely reproducible, and the cell culture container can produce a large number of uniform cellular structures.

The mass of cells produced using a known cell culture container is simply a collection of cells that cannot adhere to a culture surface that is cell non-adhesive, leading to the problem of low viability of the cells constituting the cell mass.

A cellular structure (spheroid) produced with the cell culture container according to an embodiment of the present disclosure, however, is formed through the process of cells rapidly adhering to the cell surface and expanding while growing. Between these cells, a rich extracellular matrix is produced. The cellular structure itself therefore has an extremely high viability (activity).

With this cell culture container, the experimenter can appropriately determine the size of the cellular structures in accordance with purpose and can either provide the size with a distribution or make the size uniform.

The cellular structure can be minute in size (for example, several hundred µm or less) or may have a diameter of 50 µm to 1,500 µm, with a diameter of 50 µm to 200 µm being preferable.

Since this cell culture container allows production of cellular structures on a flat plate culture surface, the culture container can be observed directly with a microscope when performing an assay on the cellular structures produced using the cell culture container.

This cell culture container also makes it possible for cells not to adhere to the outer edge of the culture surface, which is the border between the culture surface and the wall of the cell culture container, allowing uniformity in size to be maintained for an extended period of time.

To improve the effects of the present disclosure, the contact angle of water relative to the first coated regions in the cell culture container according to an embodiment of the present disclosure is preferably from 50° to 90°, more preferably 60° to 80°, and particularly preferably 62° to 78°.

The contact angle of water relative to the first coated regions refers to the average contact angle measured in conformity with JIS R3257 at several arbitrary points within the first coated regions.

As described above, the first coated regions are preferably regions with a predetermined degree or higher of hydrophobicity to increase the interaction with the cell surface and to increase the adhesiveness of cells relative to the first coated regions.

The culture surface of the cell culture container before coating with the temperature-responsive polymer or temperature-responsive polymer composition is preferably cell non-adhesive.

"Cell non-adhesive" refers to adherent cells (for example, fibroblasts, hepatocytes, vascular endothelial cells, and the like) either not adhering or tending not to adhere under normal culture conditions.

This configuration can inhibit adhesion of seeded cells to the non-coated region, i.e. the region other than the first coated regions, of the culture surface. Cells that do not adhere can be removed from the cell culture container by a simple operation, such as medium replacement. Adverse effects on the growth of the adhered cells, such as apoptosis and the production of heat shock proteins due to the non-adhering cells, can be suppressed. Making the culture surface of the cell culture container cell non-adhesive also allows production of separate cellular structures, without contact between the cells seeded in each first coated region.

Examples of the material of the cell culture container include polystyrene, polyethylene terephthalate (PET), polypropylene, polybutene, polyethylene, and polycarbonate. Polystyrene and PET are preferable for being easy to mold precisely, for allowing adoption of various sterilization methods, and for being suitable for microscope observation by virtue of being transparent.

In greater detail, since the surface zeta potential of an unprocessed cell culture container of the aforementioned polystyrene material or the like is a relatively large negative value, the negatively charged cell surface and the culture surface electrostatically repel each other. This is disadvantageous for cell adhesion. Even when using a medium containing proteins that can adhere to a polystyrene surface and change the zeta potential to be positive, the zeta potential of the surface often remains a value that is negative or near zero.

Coating a cell culture container of the aforementioned polystyrene material or the like with a temperature-responsive polymer or a temperature-responsive polymer composition causes the surface zeta potential of the polystyrene material or the like to diminish in proportion to the square of the thickness of the polymer or polymer composition (i.e. the distance from the culture surface before coating), so that the surface zeta potential of the coated culture surface is displaced further in the positive direction. Coating is thus advantageous for cell adhesion.

A typical cell culture plate is made of plastic, such as polystyrene, that is surface treated (for example, plasma treated) for cells to adhere more easily to the culture surface.

The area of the first coated region in the cell culture container is preferably from 0.1 mm² to 30 mm². Adopting this range makes it easier to obtain the desired minute spheroids.

The distance between first coated regions in the cell culture container is preferably from 0.1 mm to 10 mm.

The "distance between first coated regions" refers to the shortest distance on the culture surface between first coated regions.

Setting this distance to 0.1 mm or greater can inhibit adhesion between the cells seeded in adjacent first coated regions. Setting this distance to 10 mm or less allows efficient production of a large quantity of cellular structures (spheroids or the like).

In the cell culture container according to an embodiment of the present disclosure, the amount of temperature-responsive polymer per unit area in the first coated regions of the culture surface of the cell culture container is preferably 5.0 ng/mm² to 50 ng/mm² and more preferably 15 ng/mm² to 40 ng/mm². Adopting these ranges facilitates achievement of the effect of easier formation of cellular structures.

The number of first coated regions in the cell culture container of the present embodiment may be set appropriately in accordance with the experimenter's purpose. The number may be 2 or more, 10 or more, or 1,000 or more.

The shape of the first coated regions in planar view in the cell culture container is preferably a circle, ellipse, or another shape with the center of curvature on the inside of the outer contour line. The radius of curvature is preferably 0.1 mm to 50 mm and more preferably 1 mm to 10 mm.

The diameter of a circular first coated region is preferably 10 µm to 10 mm and more preferably 30 µm to 1,500 µm.

One of these shapes may be used alone, or a combination of two or more shapes may be used.

The culture surface of the culture cell container may be coated with the temperature-responsive polymer by methods such as precipitation from the state of an aqueous solution, application of a solution and drying of the solvent, exposure to radiation, exposure to low temperature plasma, corona discharge, glow discharge, ultraviolet light, or graft polymerization using a radical generating agent.

A method of coating the culture surface of the cell culture container with a temperature-responsive polymer or a temperature-responsive polymer composition and a method of arranging a temperature-responsive polymer or a temperature-responsive polymer composition at a plurality of positions on the culture surface that become the first coated regions are described below.

The cell culture container of the present embodiment may include only the above-described first coated regions, without including the below-described second coated regions. In this case, the cell culture container may be produced on the basis of a cell culture plate (for example, a 35 mm dish or the like), which can, for example, be used suitably in the field of regenerative medicine.

The cell culture container of the present embodiment may also include the below-described second coated regions on the inside of the above-described first coated regions. In this case, the cell culture container may be produced on the basis of a microplate (for example, a 96 hole plate or the like), which can, for example, be used suitably in the field of drug discovery (in particular, drug screening).

The cell culture container of the present embodiment may include second coated regions that are further coated with a cell adhesive material, and these second coated regions may be on the inside of the first coated regions.

Examples of the cell adhesive material include laminin, collagen, fibronectin, peptides, cationic polymers, polystyrene, and the like.

Examples of peptides include peptides containing at least one set of an arginine-glycine-aspartic acid sequence in the molecule, peptides containing a sequence of eight or more consecutive arginine molecules, and peptides containing a sequence of 10 or more consecutive lysine molecules. Examples of cationic polymers include N,N-dimethylaminopropyl acrylamide, N,N-dimethylaminoethyl methacrylamide, and aminostyrene.

Among these, laminin, collagen, and fibronectin, which have high cell adhesiveness, are preferable.

Reagents containing the above-listed cell adhesive materials can also be suitably used. Examples of such reagents include human, bovine, dog, pig, or fish serum, and a serum-free medium, which are known to a person skilled in the art.

With the cell culture container including the second coated regions, a portion of the formed cellular structures remain adhered to the second coated regions, allowing the cellular structures to be fixed to the position of each first coated region in the cell culture container.

Both the first coated regions and the second coated regions in the present disclosure are colorless and transparent, extremely thin, and flat. Light passing through either region therefore experiences nearly no refraction, reflection, or absorption, and the cell culture container can be measured (by optical measurement or the like) as is.

Seeding too few cells relative to the area of the culture surface of the cell culture container (an excessively low cell seeding density) leads to apoptosis. It follows that there is a minimum cell seeding density in order to obtain live cells.

When culturing cells with a cell culture container provided with a plurality of known wells, the number of cells to seed ends up being determined in accordance with the size of the wells.

Along with the number of cells to seed, a consideration of factors such as the necessary nutrients for each individual cell and the volume that allows physical immersion of the entire cell determines the culture scale necessary for culturing the cellular structures. The amount of medium required for culturing and the amount of a drug used in a test are also thereby determined.

The desire to perform experiments such as iPS cell experiments efficiently on a small scale has increased in this technical field in recent years. Experimental costs may not be sufficiently reduced even when using the smallest cell culture container, with 384 wells, among current international standardized products.

A cell culture container including the second coated regions of the present embodiment allows numerous cellular structures (spheroids) to exist in the same space without being separated into independent spaces by partitions or the like. Differentiation inducing factors used to differentiate cellular structures, drugs to be screened, and the like can therefore be included in a medium, and the medium can simply be added at once (stirring as necessary) to a system in which numerous cellular structures are also present to place all of the cellular structures under the same conditions. Physiologically active substances secreted by the cellular structures can also be dispersed uniformly in the system.

The area of each second coated region in the cell culture container is preferably from 0.001 mm² to 10 mm². Adopting this range allows a balanced achievement of the effect, due to the above-described first coated regions, of forming the desired minute spheroids and the effect, due to the above-described second coated regions, of fixing the cellular structures to the cell culture container.

In the cell culture container, the amount of cell adhesive material per unit area in the second coated regions is preferably 1.0 ng/mm² to 1,000 ng/mm² and more preferably 10 ng/mm² to 100 ng/mm². Adopting these ranges allows efficient formation and fixing of the cellular structures.

The number of second coated regions inside the first coated regions may be set appropriately in accordance with the experimenter's purpose. One second coated region, or two or more, may be provided in each first coated region.

The shape of the second coated regions in planar view in the cell culture container is preferably a circle, ellipse, or another shape with the center of curvature on the inside of the outer contour line. The radius of curvature is preferably 0.1 mm to 50 mm and more preferably 1 mm to 10 mm.

The diameter of a circular second coated region is preferably 0.01 mm to 10 mm and more preferably 10 µm to 1,000 µm.

One of these shapes may be used alone, or a combination of two or more shapes may be used.

The temperature-responsive polymer and temperature-responsive polymer composition used in the first coated regions of the cell culture container according to an embodiment of the present disclosure are now described in detail.

Examples of the temperature-responsive polymer and temperature-responsive polymer composition used in the cell culture container according to an embodiment of the present disclosure include (A) a temperature-responsive polymer containing 2-N,N-dimethylaminoethyl methacrylate (DMAEMA) units and anionic monomer units, (B) a temperature-responsive polymer containing N-isopropyl acrylamide (NIPAM) units, cationic monomer units, and anionic monomer units, and (C) a temperature-responsive polymer composition containing a polymer of 2-N,N-dimethylaminoethyl methacrylate (DMAEMA) and/or a derivative thereof, 2-amino-2-hydroxymethyl-1,3-propanediol (tris), and one or more anionic substances selected from the group consisting of nucleic acids, heparin, hyaluronic acid, dextran sulfate, polystyrene sulfonic acid, polyacrylic acid, polymethacrylic acid, polyphosphoric acid, sulfated polysaccharide, curdlan, polyarginic acid, and alkali metal salts thereof.

Examples of (A) include (A-1) a temperature-responsive polymer obtained by a method for polymerizing DMAEMA in the presence of water and (A-2) a temperature-responsive polymer containing a polymer block principally containing DMAEMA (polymer chain α terminal) and principally containing DMAEMA and an anionic monomer (polymer chain ω terminal).

One type these may be used alone, or a combination of two or more types may be used in the present embodiment.

The temperature-responsive polymer of (A-1) and a manufacturing method thereof are described below.

### (Manufacturing method of temperature-responsive polymer)

In a manufacturing method of the temperature-responsive polymer of (A-1), a mixture containing 2-N,N-dimethylaminoethyl methacrylate (DMAEMA) is first prepared (preparation step). The mixture further includes a polymerization inhibitor and water.

A commercial product may be used as the 2-N,N-dimethylaminoethyl methacrylate (DMAEMA). Examples of the polymerization inhibitor include methylhydroquinone (MEHQ), hydroquinone, p-benzoquinoline, N,N-diethylhydroxylamine, N-nitroso-N-phenylhydroxylamine (Cupferron), and t-butylhydroquinone. MEHQ or the like included in commercially available DMAEMA may be used as is. Examples of water include ultrapure water.

The weight ratio of the polymerization inhibitor to the mixture is preferably 0.01% to 1.5% and more preferably 0.1% to 0.5%. Adopting these ranges suppresses a runaway radical polymerization reaction and reduces the occurrence of uncontrollable crosslinking, while also providing the manufactured temperature-responsive polymer with solubility in a solvent.

The weight ratio of the water to the mixture is preferably 1.0% to 50% and more preferably 9.0% to 33%. Adopting these ranges achieves a good balance between the reaction rate of the hydrolysis reaction of the side chain and the reaction rate of the growth reaction of the polymer chain being polymerized. It is thus possible to obtain a temperature-responsive polymer having a ratio of DMAEMA in which the side chain is not hydrolyzed to DMAEMA in which the side chain is hydrolyzed (the copolymerization ratio) of approximately 1.0 to 20.

Next, in the manufacturing method of the temperature-responsive polymer of (A-1), the mixture is irradiated with ultraviolet light (irradiation step). Here, the irradiation with ultraviolet light takes place under an inert atmosphere. The DMAEMA undergoes radical polymerization by irradiation with ultraviolet light to become a polymer.

In this step, the aforementioned mixture is added to a transparent, sealed vial, for example, and an inert atmosphere is formed inside the vial by bubbling an inert gas. Subsequently, the mixture is irradiated with ultraviolet light from outside the vial using an ultraviolet light irradiation device.

The wavelength of the ultraviolet light is preferably 210 nm to 600 nm and more preferably 360 nm to 380 nm. These wavelength ranges can cause the polymerization reaction to progress efficiently and stably yield polymer material with the desired copolymerization ratio. These wavelength ranges can also prevent coloring of the manufactured polymer material.

Examples of the inert gas include nitrogen, argon, helium, and neon.

Among reaction conditions, the temperature condition is preferably from 15°C to 50°C, more preferably from 20°C to 30°C. These temperature ranges suppress a heat initiated reaction, giving preference instead to an initiation reaction due to irradiation with light. Furthermore, the reaction rates of the hydrolysis reaction can be balanced well against the reaction rate of the growth reaction of the polymer chain.

The reaction time is preferably from 7 hours to 24 hours, more preferably from 17 hours to 21 hours. These time ranges can obtain a high yield of the temperature-responsive polymer of (A-1) and allow radical polymerization while suppressing a photolytic reaction and an unnecessary crosslinking reaction.

The time from when preparation of the mixture in the preparation step is finished until the start of irradiation with ultraviolet light in the irradiation step is preferably from 10 minutes to 1 hour.

It takes approximately 10 minutes to replace the gas inside a vial to which the mixture is added and to form an inert atmosphere inside the vial. Setting the aforementioned time to less than 10 minutes may therefore not result in the inert atmosphere necessary for radical polymerization. On the other hand, the hydrolysis reaction of DMAEMA in the mixture starts before the start of irradiation with ultraviolet light. Setting the aforementioned time to longer than one hour therefore yields a large amount of methacrylic acid, which is inactive in the radical polymerization reaction, in the mixture.

In the manufacturing method of the temperature-responsive polymer of (A-1), water is included in the mixture. The radical polymerization reaction of DMAEMA and the hydrolysis reaction of the ester bond in the side chain of the poly(2-N,N-dimethylaminoethyl methacrylate) (PDMAEMA) can therefore be caused to compete.

The product yielded by this competition is a polymer including the repeating unit (A) represented by formula (I), 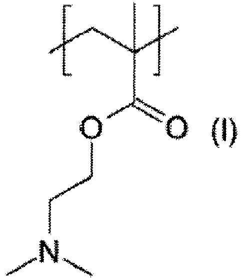 and the repeating unit (B) represented by formula (II).

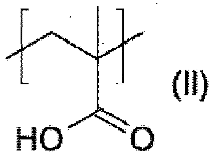

Therefore, a good balance of both a cationic functional group included in a polymer, i.e. a dimethylamino group, and an anionic functional group included in a polymer, i.e. a carboxyl group formed by hydrolysis of the ester bond in a side chain, can be provided. The manufacturing method of the temperature-responsive polymer of (A-1) can then easily produce, with few steps, a polymer derived from poly(2-N,N-dimethylaminoethyl methacrylate) and including a cationic functional group and an anionic functional group.

Even without using the same manufacturing method as the manufacturing method of the temperature-responsive polymer of (A-1), the same effects as those of the manufacturing method of a temperature-responsive polymer according to the present disclosure may be obtained if DMAEMA, a polymerization inhibitor, and water are present together in the reaction system at the time of irradiating with ultraviolet light.

For example, the following manufacturing method of a temperature-responsive polymer can also be used for the temperature-responsive polymer of (A-1): water and a mixture containing DMAEMA and a polymerization inhibitor are prepared separately, an inert gas is then bubbled in the mixture and the water, and subsequently, the mixture and the water are mixed under an inert atmosphere while simultaneously being irradiated with ultraviolet light.

### (Temperature-responsive polymer)

The temperature-responsive polymer of (A-1) is manufactured by the aforementioned manufacturing method of (A-1).

The temperature-responsive polymer of (A-1) is preferably a molecule with a number-average molecular weight (Mn) of 10 kDa to 500 kDa. The temperature-responsive polymer of (A-1) is also preferably a molecule for which the ratio (Mw/Mn) of the weight-average molecular weight (Mw) to the number-average molecular weight (Mn) is 1.1 to 10.0.

The molecular weight of the temperature-responsive polymer of (A-1) can be appropriately adjusted by the conditions on the irradiation time and irradiation intensity of the ultraviolet light.

According to the temperature-responsive polymer of (A-1), the cloud point can be reduced, for example to room temperature (25°C) or below.

Insoluble matter of the temperature-responsive polymer (A-1) formed at a temperature at or above the cloud point exhibits an extremely long delay until becoming soluble again at room temperature (approximately 25°C). The reason is thought to be that the resulting temperature-responsive polymer of (A-1) has high self-cohesion due to the presence of a cationic functional group and an anionic functional group in the molecule.

As described below, the temperature-responsive polymer of (A-1) can be used to prepare a cell culture container having a culture surface coated with this temperature-responsive polymer.

Furthermore, as described below, the temperature-responsive polymer of (A-1) allows formation of cellular structures that have a luminal (tube-like) and an aggregated (pellet-like) structure by culturing cells under appropriate culture conditions.

The ratio (C/A ratio) of the number of cationic functional groups (2-N,N-dimethylamino groups) to the number of anionic functional groups (carboxyl groups) in the temperature-responsive polymer of (A-1) is preferably from 0.5 to 32 and more preferably from 4 to 16.

Setting the C/A ratio in these ranges facilitates achievement of the aforementioned effect of reducing the cloud point. The reason is thought to be that in a temperature-responsive polymer with the aforementioned C/A ratio, the cationic functional group and the anionic functional group in the temperature-responsive polymer affect inter- and/or intra-molecular aggregation by ionic bonding, thereby increasing the aggregation strength of the temperature-responsive polymer.

Another reason is thought to be that setting the C/A ratio within the aforementioned ranges can suppress cytotoxicity due to positive charges by achieving a particularly preferable balance between positive and negative charges in the temperature-responsive polymer and can also facilitate cell migration and orientation by achieving a particularly preferable balance between hydrophilicity and hydrophobicity of the temperature-responsive polymer.

The temperature-responsive polymer of (A-2) and a manufacturing method thereof are described below.

### (Manufacturing method of temperature-responsive polymer)

In a manufacturing method of the temperature-responsive polymer of (A-2), a first mixture containing 2-N,N-dimethylaminoethyl methacrylate (DMAEMA) is first irradiated with ultraviolet light (first polymerization step).

Other than DMAEMA, the first mixture may, for example, optionally include another monomer, solvent, or the like.

The irradiation with ultraviolet light may take place under an inert atmosphere.

A commercially available product may be used for the DMAEMA.

Examples of the other monomers that may be included in the first mixture include, for example, N,N-dimethyl acrylamide, esters of acrylic acid or methacrylic acid having polyethylene glycol side chains, N-isopropyl acrylamide, 3-N,N-dimethylaminopropyl acrylamide, and 2-N,N-dimethylaminoethyl methacrylamide. In particular, N,N-dimethyl acrylamide, esters of acrylic acid or methacrylic acid having polyethylene glycol side chains, and N-isopropyl acrylamide are preferable for allowing the ion balance to be adjusted stably. One type these monomers may be used alone, or a combination of two or more types may be used. The ratio (number of moles) of the amount of other monomers used to the amount of DMAEMA used is preferably from 0.001 to 1 and more preferably from 0.01 to 0.5.

Examples of the solvent include toluene, benzene, chloroform, methanol, and ethanol. In particular, toluene and benzene are preferable by virtue of being inert relative to the ester bond of the DMAEMA. One type these solvents may be used alone, or a combination of two or more types may be used.

In this step, the aforementioned first mixture is added to a transparent, sealed vial, for example, and an inert atmosphere is formed inside the vial by bubbling an inert gas. Subsequently, the first mixture is irradiated with ultraviolet light from outside the vial using an ultraviolet light irradiation device.

The wavelength of the ultraviolet light is preferably 210 nm to 600 nm and more preferably 360 nm to 380 nm. These wavelength ranges cause the polymerization reaction to progress efficiently and stably yield polymer material with the desired copolymerization ratio. These wavelength ranges can also prevent coloring of the manufactured polymer material.

The irradiation intensity of the ultraviolet light is preferably from 0.01 mW/cm² to 50 mW/cm² and more preferably from 0.1 mW/cm² to 5 mW/cm². These ranges of irradiation intensity can suppress decomposition due to unnecessary cutting of chemical bonds or the like while stably allowing the polymerization reaction to proceed at an appropriate rate (time).

Examples of the inert gas include nitrogen, argon, helium, and neon.

The temperature condition is preferably from 10°C to 40°C, more preferably from 20°C to 30°C. These temperature ranges allow the reaction to take place at room temperature in a typical laboratory while suppressing a reaction due to means other than light (such as heat).

The reaction time is preferably from 10 minutes to 48 hours, more preferably from 60 minutes to 24 hours.

In this step, the DMAEMA undergoes radical polymerization by irradiation with the ultraviolet light and becomes a polymer (poly(2-N,N-dimethylaminoethyl methacrylate), i.e. PDMAEMA), thereby forming a homopolymer block containing 2-N,N-dimethylaminoethyl methacrylate. In the case of also using another monomer, a polymer block containing DMAEMA and the other monomer is formed.

Next, in the manufacturing method of the temperature-responsive polymer of (A-2), at the point when the number-average molecular weight of a polymer (specifically, polymerized 2-N,N-dimethylaminoethyl methacrylate) reaches at least a predetermined value in the first polymerization step, an anionic monomer is added to the first mixture to prepare a second mixture (adding step).

Other than the first mixture after the first polymerization step and the anionic monomer, the second mixture may, for example, include another monomer, the above-described solvents that can be included in the first mixture (such as toluene, benzene, or methanol), and the like.

The anionic monomer may be added under an inert atmosphere.

Examples of the anionic monomer include acrylic acid, methacrylic acid, vinyl derivatives containing at least one group selected from the group consisting of a carboxyl group, a sulfonic acid group, and a phosphoric acid group. In particular, acrylic acid and methacrylic acid in a side chain are preferable in terms of chemical stability.

One type these anionic monomers may be used alone, or a combination of two or more types may be used.

Examples of the other monomers that may be included in the second mixture include, for example, N,N-dimethyl acrylamide, esters of acrylic acid or methacrylic acid having polyethylene glycol side chains, N-isopropyl acrylamide, 3-N,N-dimethylaminopropyl acrylamide, and 2-N,N-dimethylaminoethyl methacrylamide. N,N-dimethyl acrylamide, which is electrically neutral and hydrophilic, is particularly preferable. One type these monomers may be used alone, or a combination of two or more types may be used. The ratio (in moles) of the amount of other monomers used to the amount of DMAEMA used is preferably from 0.01 to 10 and more preferably from 0.1 to 5.

In this step, the second mixture is added while, for example, maintaining an inert atmosphere in the vial by causing an inert gas to flow into the vial.

The predetermined value of the number-average molecular weight is preferably 5,000, more preferably 20,000, and particularly preferably 100,000 to sufficiently obtain the effect of reducing the cloud point.

The number-average molecular weight of the polymerized PDMAEMA in the first mixture after the first polymerization step can be measured by sampling a small amount of the reaction mixture from the polymerization system at a predetermined point in time and using a method known to a person skilled in the art, such as gel permeation chromatography (GPC) or static light scattering (SLS).

In this step, an anionic monomer is included in the polymerization system in addition to the homopolymer containing DMAEMA that is being polymerized. The polymerization system in the vial thereby changes from a homopolymerization system of DMAEMA to a copolymerization system of DMAEMA and an anionic monomer.

In the manufacturing method of the temperature-responsive polymer of (A-2), the second mixture is then irradiated with ultraviolet light (second polymerization step).

Here, the irradiation with ultraviolet light may take place under an inert atmosphere.

During this step, the vial to which the second mixture has been added is, for example, irradiated with ultraviolet light from outside using an ultraviolet light irradiation device.

The conditions in the second polymerization step, such as the wavelength of the ultraviolet light, the radiation intensity of the ultraviolet light, the inert gas that is used, the reaction temperature, and the reaction time may be the same as the conditions in the first polymerization step.

In this step, the DMAEMA and the anionic monomer undergo radical polymerization by irradiation with the ultraviolet light, and a copolymer block containing DMAEMA and the anionic monomer is formed to be continuous with the polymer chain α terminal of the homopolymer block, which includes DMAEMA, formed in the first polymerization step. In the case of also using another monomer, a copolymer block containing DMAEMA, an anionic monomer, and the other monomer is formed.

As described above, a temperature-responsive polymer containing a homopolymer block containing DMAEMA and a copolymer block of DMAEMA and an anionic monomer is obtained.

As will be understood by a person skilled in the art, while mixtures of polymers having various molecular weights and molecular structures are produced with the manufacturing method of (A-2), polymerization is preferably carried out under identical conditions throughout the first polymerization step, the adding step, and the second polymerization step to obtain, as the principal component, a temperature-responsive polymer containing a homopolymer block containing DMAEMA and a copolymer block of DMAEMA and an anionic monomer.

### (Temperature-responsive polymer)

The temperature-responsive polymer of (A-2) is manufactured by the aforementioned manufacturing method of (A-2).

The temperature-responsive polymer of (A-2) contains a polymer block (polymer chain α terminal) principally containing 2-N,N-dimethylaminoethyl methacrylate and optionally containing other monomer units such as dimethyl acrylamide, acrylic acid or methacrylic acid having polyethylene glycol side chains, or another such hydrophilic monomer; and contains a copolymer block principally containing 2-N,N-dimethylaminoethyl methacrylate and an anionic monomer (polymer chain ω terminal) and optionally containing other monomer units.

The temperature-responsive polymer of (A-2) preferably contains a homopolymer block of DMAEMA and a copolymer block of DMAEMA and an anionic monomer, and the temperature-responsive polymer of (A-2) is more preferably composed of these blocks.

As the temperature-responsive polymer of (A-2), the number-average molecular weight of the polymer block of the polymer chain α terminal (for example, the homopolymer block of DMAEMA) is preferably 5,000 Da or greater and more preferably 20,000 Da or greater.

The temperature-responsive polymer of (A-2) is preferably a molecule with a number-average molecular weight (Mn) of 10 kDa to 500 kDa. The temperature-responsive polymer of (A-2) is also preferably a molecule for which the ratio (Mw/Mn) of the weight-average molecular weight (Mw) to the number-average molecular weight (Mn) is 1.1 to 10.0.

The molecular weight of the temperature-responsive polymer can be appropriately adjusted by the conditions on the irradiation time and irradiation intensity of the ultraviolet light.

According to the temperature-responsive polymer of (A-2), the cloud point can be reduced, for example to room temperature (25°C) or below.

Insoluble matter of the temperature-responsive polymer (A-2) formed at a temperature at or above the cloud point exhibits an extremely long delay until becoming soluble again at room temperature (approximately 25°C). The reason is thought to be that the resulting temperature-responsive polymer has high self-cohesion due to the presence of a cationic functional group and an anionic functional group in the molecule.

In particular, it is thought that since the temperature-responsive polymer of (A-2) includes a homopolymer block of DMAEMA having a high molecular weight (such as 5,000 Da or greater) at the polymer chain α terminal, temperature dependent globule transition of the side chain of DMAEMA occurs more easily, effectively reducing the cloud point.

As described below, this temperature-responsive polymer can be used to prepare a cell culture container having a culture surface coated with this temperature-responsive polymer.

Furthermore, as described below, the temperature-responsive polymer of (A-2) allows formation of cellular structures that have an aggregated (pellet-like) structure by culturing cells under appropriate culture conditions.

The ratio (C/A ratio) of the number of cationic functional groups (2-N,N-dimethylamino groups) to the number of anionic functional groups (carboxyl groups) in the temperature-responsive polymer of (A-2) is preferably from 0.5 to 32 and more preferably from 4 to 16.

Setting the C/A ratio in these ranges facilitates achievement of the aforementioned effect of reducing the cloud point. The reason is thought to be that in a temperature-responsive polymer with the aforementioned C/A ratio, the cationic functional group and the anionic functional group in the temperature-responsive polymer affect inter- and/or intra-molecular aggregation by ionic bonding, thereby increasing the aggregation strength of the temperature-responsive polymer.

Another reason is thought to be that setting the C/A ratio within the aforementioned ranges can suppress cytotoxicity due to positive charges by achieving a particularly preferable balance between positive and negative charges in the temperature-responsive polymer and can also facilitate cell migration and orientation by achieving a particularly preferable balance between hydrophilicity and hydrophobicity of the temperature-responsive polymer.

The temperature-responsive polymer of (B) and a manufacturing method thereof are described below.

### (Manufacturing method of temperature-responsive polymer)

A manufacturing method of the temperature-responsive polymer of (B) polymerizes N-isopropyl acrylamide (NIPAM) (monomer (A)), a cationic monomer (monomer (B)), and an anionic monomer (monomer (C)). Another monomer, other than the aforementioned three types of monomers, may optionally be added and polymerized.

A commercially available product may be used for the N-isopropyl acrylamide (NIPAM).

Examples of cationic monomers include monomers having a cationic functional group. Examples of cationic functional groups include amino groups, such as primary to quaternary amino groups, and guanidine groups. In particular, tertiary amino groups are preferable for chemical stability, low cytotoxicity, sterilization stability, and a strong positive charge.

More specifically, the cationic monomer is preferably very stable even when supporting a physiologically active substance or under alkaline conditions. Examples include 3-(N,N-dimethylaminopropyl)-(meth)acrylamide, 3-(N,N-dimethylaminopropyl)-(meth)acrylate, aminostyrene, 2-(N,N-dimethylaminoethyl)-(meth)acrylamide, and 2-(N,N-dimethylaminoethyl)-(meth)acrylate.

Among these, 3-(N,N-dimethylaminopropyl)acrylamide is particularly preferable for easily supporting an anionic substance by virtue of having a strong positive charge.

Aminostyrene is also preferable for easily supporting an anionic substance by virtue of having a strong positive charge while also increasing the number of variations of supportable anionic substances through interaction between an aromatic ring in the molecule and a hydrophobic structure of another substance in the aqueous solution.

Furthermore, 2-(N,N-dimethylaminoethyl)-methacrylamide is preferable for having a weak positive charge at a neutral pH and for its solubility in water not being affected by temperature, thereby allowing easy ejection of an anionic substance that has been supported once.

One type these cationic monomers may be used alone, or a combination of two or more types may be used.

Examples of anionic monomers include monomers having an anionic functional group. Examples of anionic functional groups include a carboxylic acid group, a sulfonic acid group, a sulfuric acid group, a phosphoric acid group, and a boronic acid group. In particular, a carboxylic acid group, a sulfonic acid group, and a phosphoric acid group are preferable for chemical stability, cell affinity, and a high degree of purification.

More specifically, examples include acrylic acid, methacrylic acid, and vinylbenzoic acid. In particular, methacrylic acid and vinylbenzoic acid are preferable for chemical stability and cell affinity.

One type these anionic monomers may be used alone, or a combination of two or more types may be used.

Examples of other monomers include dimethyl acrylamide, acrylic acid or methacrylic acid having polyethylene glycol side chains, or another such neutral hydrophilic monomer.

One type these monomers may be used alone, or a combination of two or more types may be used.

The other monomers can be used to adjust the hydrophilic/hydrophobic balance apart from charge and can increase the number of variations.

Taking into consideration the reactivity in the polymerization reaction of the monomers, a person skilled in the art can appropriately adjust the ratio (moles) of the amount of NIPAM used, the amount of cationic monomers used, and the amount of other monomers used relative to the total amount of monomers (A) to (C) used in the manufacturing method of the temperature-responsive polymer of (B) so that the desired ratio of monomer components is obtained.

Examples of polymerization methods include radical polymerization and ionic polymerization.

Living radical polymerization is preferable as a type of radical polymerization. Examples of living radical polymerization include reversible addition fragmentation chain transfer polymerization (RAFT), atom transfer radical polymerization (ATRP), and iniferter polymerization, with iniferter polymerization being preferable.

Living anionic polymerization is preferable as ionic polymerization.

An example of the manufacturing method of the temperature-responsive polymer of (B) is a method using radical polymerization.

In this example of a manufacturing method, a first mixture containing N-isopropyl acrylamide (NIPAM) is first irradiated with ultraviolet light (first polymerization step).

Other than DMAEMA, the first mixture may, for example, optionally include another monomer, a solvent, a chain transfer agent, a stabilizer, a surfactant, or the like.

The irradiation with ultraviolet light may take place under an inert atmosphere.

In this step, the aforementioned first mixture is added to a transparent, sealed vial, for example, and an inert atmosphere is formed inside the vial by bubbling an inert gas. Subsequently, the first mixture is irradiated with ultraviolet light from outside the vial using an ultraviolet light irradiation device.

Examples of the solvent include benzene, toluene, chloroform, methanol, and water. In particular, benzene and toluene are preferable in terms of solubility and for being inert during polymerization. One type these solvents may be used alone, or a combination of two or more types may be used.

In this step, the aforementioned first mixture is added to a transparent, sealed vial, for example, and an inert atmosphere is formed inside the vial by bubbling an inert gas. Subsequently, the first mixture is irradiated with ultraviolet light from outside the vial using an ultraviolet light irradiation device.

The wavelength of the ultraviolet light is preferably 210 nm to 600 nm and more preferably 360 nm to 380 nm. These wavelength ranges cause the polymerization reaction to progress efficiently and stably yield polymer material with the desired copolymerization ratio. These wavelength ranges can also prevent coloring of the manufactured polymer material.

The irradiation intensity of the ultraviolet light is preferably from 0.01 mW/cm² to 50 mW/cm² and more preferably from 0.1 mW/cm² to 5 mW/cm².

Examples of the inert gas include nitrogen, argon, helium, and neon.

The temperature condition is preferably from 10°C to 40°C, more preferably from 20°C to 30°C. These temperature ranges allow the polymerization reaction to take place at room temperature in a typical laboratory while suppressing a reaction due to means, such as heat, differing from the light irradiation means.

The reaction time is preferably from 10 minutes to 48 hours, more preferably from 60 minutes to 24 hours.

During this step, the NIPAM undergoes radical polymerization by irradiation with the ultraviolet light and becomes a polymer (poly(N-isopropyl acrylamide), i.e. PNIPAM), thereby forming a homopolymer block containing N-isopropyl acrylamide. In the case of also using another monomer, a polymer block containing NIPAM and the other monomer is formed.

Next, in the manufacturing method of the temperature-responsive polymer of (B), a second mixture is prepared by adding a cationic monomer and an anionic monomer to the first mixture after the first polymerization step (adding step).

Other than the first mixture after the first polymerization step, the cationic monomer, and the anionic monomer, the second mixture may, for example, include another monomer, a solvent, a chain transfer agent, a stabilizer, a surfactant, or the like.

The cationic monomer and the anionic monomer may be added under an inert atmosphere.

In this step, the cationic monomer and the anionic monomer are added while, for example, maintaining an inert atmosphere in the vial by causing an inert gas to flow into the vial.

In this step, a cationic monomer and an anionic monomer are included in the polymerization system in addition to the homopolymer containing NIPAM that is being polymerized. The polymerization system in the vial thereby changes from a homopolymerization system of NIPAM to a copolymerization system of NIPAM, a cationic monomer, and an anionic monomer.

In the manufacturing method of the temperature-responsive polymer of (B), the second mixture is then irradiated with ultraviolet light (second polymerization step).

Here, the irradiation with ultraviolet light may take place under an inert atmosphere.

During this step, the vial to which the cationic monomer and the anionic monomer have been added is, for example, irradiated with ultraviolet light from outside using an ultraviolet light irradiation device.

The wavelength of the ultraviolet light is preferably 210 nm to 600 nm and more preferably 360 nm to 380 nm. These wavelength ranges cause the polymerization reaction to progress efficiently and stably yield polymer material with the desired copolymerization ratio. These wavelength ranges can also prevent coloring of the manufactured polymer material.

The irradiation intensity of the ultraviolet light is preferably from 0.01 mW/cm² to 50 mW/cm² and more preferably from 0.1 mW/cm² to 5 mW/cm².

Examples of the inert gas include nitrogen, argon, helium, and neon.

The temperature condition is preferably from 10°C to 40°C, more preferably from 20°C to 30°C. These temperature ranges allow the polymerization reaction to take place at room temperature in a typical laboratory while suppressing a reaction due to means, such as heat, differing from the light irradiation means.

The reaction time is preferably from 10 minutes to 48 hours, more preferably from 60 minutes to 24 hours.

In this step, the NIPAM, the cationic monomer, and the anionic monomer undergo radical polymerization by irradiation with the ultraviolet light, and a copolymer block containing NIPAM, the cationic monomer, and the anionic monomer is formed to be continuous with the polymer chain α terminal of the homopolymer block, which includes NIPAM, formed in the first polymerization step. In the case of also using another monomer, a polymer block containing NIPAM and the other monomer, and/or a copolymer block containing NIPAM, the cationic monomer, the anionic monomer, and the other monomer is formed.

As described above, a temperature-responsive polymer containing a homopolymer block containing NIPAM and a copolymer block of NIPAM, a cationic monomer, and an anionic monomer is obtained.

In this example of a manufacturing method, irradiation with ultraviolet light is preferably performed throughout the first polymerization step, the adding step, and the second polymerization step to achieve an efficient reaction.

Another example of a manufacturing method of the temperature-responsive polymer of (B) is a method using radical polymerization. A mixture containing N-isopropyl acrylamide (NIPAM), a cationic monomer, and an anionic monomer, and optionally containing another monomer, is irradiated with ultraviolet light.

This mixture may, for example, include a solvent, a chain transfer agent, a stabilizer, a surfactant, or the like.

The irradiation with ultraviolet light may take place under an inert atmosphere.

Other conditions may be the same as in the above-described example of a manufacturing method.

Furthermore, in the case of using iniferter polymerization, benzyl-(N,N-diethyl)dithiocarbamate may be used as an iniferter and toluene or the like used as a solvent. Living polymerization may then be carried out by irradiation with near ultraviolet light. Here, after polymerization by a first monomer, polymerization by a second monomer can be performed after an isolation operation, thereby yielding a block copolymer.

Furthermore, in the case of using ionic polymerization, an NaOH powder may be used as the catalyst, and a solvent for reprecipitation used for purification may be used along with an aprotic solvent as the solvent. After polymerization by a first monomer, polymerization by a second monomer can be performed after a reprecipitation operation (with an ionic species remaining on the ω terminal after this operation), thereby yielding a block copolymer.

### (Temperature-responsive polymer)

The temperature-responsive polymer of (B) is manufactured by the aforementioned manufacturing method of (B).

The temperature-responsive polymer of (B) contains N-isopropyl acrylamide (NIPAM) units, cationic monomer units, and anionic monomer units, and optionally contains other monomer units. This polymer can be manufactured by the above-described example and other example of a manufacturing method.

The temperature-responsive polymer of (B) preferably contains a polymer block (polymer chain α terminal) principally containing N-isopropyl acrylamide (NIPAM) units and optionally containing other monomer units and a copolymer block principally containing cationic monomer units and anionic monomer units and optionally containing other monomer units. The temperature-responsive polymer of (B) more preferably contains a homopolymer block of NIPAM and a copolymer block of NIPAM, a cationic monomer, and an anionic monomer. The temperature-responsive polymer of (B) is particularly preferably composed of these blocks. This polymer can be manufactured by the above-described example of a manufacturing method.

For example, in the temperature-responsive polymer of PTL 1, the DMAEMA that provides the polymer with temperature responsiveness is, at the same time, a cationic monomer that is necessary for forming a cellular structure (along with an anionic monomer), and the DMAEMA involved in temperature responsiveness is included in the polymer chain α terminal as a polymer block.

Since a cationic monomer always exists in the polymer chain α terminal in this temperature-responsive polymer, the degree of freedom for adjusting the position of the cationic sites in the polymer chain is not high, and the cationic monomer is mainly limited to DMAEMA. For these reasons, it is not necessarily easy to adjust the positive charge strength of the cationic site or the pH of the temperature-responsive polymer aqueous solution.

For example, in the case of using the temperature-responsive polymer in drug delivery (DDS), the type and amount of the supportable drug may be restricted. Examples of DDS methods include a method for sustained release of a drug from a coated material to cells or tissue by applying a temperature-responsive polymer supporting a drug on a cell culture container, and then culturing cells and tissue in the cell culture container after the application. Since the temperature-responsive polymer of PTL 1 includes DMAEMA with a small positive charge strength, a drug that is an anionic substance cannot always be supported easily. Hence, the type and amount of supportable drugs may be restricted.

In contrast, in the temperature-responsive polymer of (B), the NIPAM that provides the polymer with temperature responsiveness is a neutral monomer, and the cationic monomer that is necessary for forming a cellular structure (along with an anionic monomer) is a different monomer than NIPAM.

In the temperature-responsive polymer of (B), a cationic monomer is not necessarily present at the polymer chain α terminal, and the position of the cationic site in the polymer chain can be adjusted freely. A wide range of cationic monomers can also be used. The positive charge strength of the cationic site and the pH of the temperature-responsive polymer aqueous solution can easily be adjusted.

When, for example, used in drug delivery (DDS), the temperature-responsive polymer of (B) can expand the variety of supportable drugs while also increasing the amount thereof. The temperature-responsive polymer thus has a wider range of applications.

In the temperature-responsive polymer of (B), the ratio (moles) of NIPAM units to the total of NIPAM units, cationic monomer units, and anionic monomer units is preferably from 0.6 to 0.9, is more preferably from 0.7 to 0.9, and is particularly preferably 0.9.

When also using another monomer, the ratio (moles) of the other monomer units to the total of NIPAM units, cationic monomer units, and anionic monomer units is preferably from 0.001 to 0.2 and is more preferably from 0.01 to 0.1.

As the temperature-responsive polymer of (B), the number-average molecular weight of the polymer block of the polymer chain α terminal (for example, the homopolymer block of NIPAM) is preferably 5,000 Da or greater and more preferably 20,000 Da or greater.

The temperature-responsive polymer of (B) is preferably a molecule with a number-average molecular weight (Mn) of 10 kDa to 500 kDa. The temperature-responsive polymer of (B) is also preferably a molecule for which the ratio (Mw/Mn) of the weight-average molecular weight (Mw) to the number-average molecular weight (Mn) is 1.1 to 10.0.

The molecular weight of the temperature-responsive polymer can be appropriately adjusted by the polymerization conditions.

According to the temperature-responsive polymer of (B), the cloud point can be reduced, for example to room temperature (25°C) or below.

Insoluble matter of the temperature-responsive polymer formed at a temperature at or above the cloud point exhibits an extremely long delay until becoming soluble again at room temperature (approximately 25°C). The reason is thought to be that the resulting temperature-responsive polymer has high self-cohesion due to the presence of a cationic functional group and an anionic functional group in the molecule.

In particular, it is thought that since the temperature-responsive polymer of (B) includes a homopolymer block of NIPAM having a high molecular weight at the polymer chain α terminal, temperature dependent globule transition of the side chain of NIPAM occurs more easily, effectively reducing the cloud point.

As described below, this temperature-responsive polymer can be used to prepare a cell culture container having a culture surface coated with this temperature-responsive polymer.

Furthermore, as described below, the temperature-responsive polymer of (B) allows formation of cellular structures that have a luminal (tube-like) or an aggregated (pellet-like) structure by culturing cells under appropriate culture conditions.

The ratio (C/A ratio) of the number of cationic functional groups to the number of anionic functional groups in the temperature-responsive polymer of (B) is preferably from 0.5 to 32 and more preferably from 4 to 16.

Setting the C/A ratio in these ranges facilitates achievement of the aforementioned effect of reducing the cloud point. The reason is thought to be that in a temperature-responsive polymer with the aforementioned C/A ratio, the cationic functional group and the anionic functional group in the temperature-responsive polymer affect inter- and/or intra-molecular aggregation by ionic bonding, thereby increasing the aggregation strength of the temperature-responsive polymer.

Another reason is thought to be that setting the C/A ratio within the aforementioned ranges can suppress cytotoxicity due to positive charges by achieving a particularly preferable balance between positive and negative charges in the temperature-responsive polymer and can also facilitate cell migration and orientation by achieving a particularly preferable balance between hydrophilicity and hydrophobicity of the temperature-responsive polymer.

The temperature-responsive polymer of (C) and a manufacturing method thereof are described below.

### (Manufacturing method of temperature-responsive polymer composition)

In a manufacturing method of a temperature-responsive polymer composition of (C), a mixed-type temperature-responsive polymer composition is first prepared (mixture preparation step). Specifically, (1) a polymer of 2-N,N-dimethylaminoethyl methacrylate (DMAEMA) and/or a derivative thereof, (2) 2-amino-2-hydroxymethyl-1,3-propanediol (tris), (3) and one or more anionic substances selected from the group consisting of nucleic acids, heparin, hyaluronic acid, dextran sulfate, polystyrene sulfonic acid, polyacrylic acid, polymethacrylic acid, polyphosphoric acid, sulfated polysaccharide, curdlan, polyarginic acid, and alkali metal salts thereof are mixed ((2) tris being optionally included).

The (1) polymer of DMAEMA and/or a derivative thereof is a temperature-responsive polymer with a cloud point of 32°C. It is inferred that the (2) tris has the function of slightly reducing the cloud point and/or reducing the speed at which a polymer formed at a higher temperature than the cloud point becomes soluble again when cooled to the cloud point or lower. It is also inferred that the (2) tris has the function of stimulating cells by a positive charge derived from an amino group while maintaining hydrophilicity even in a hydrophobized polymer layer. It is inferred that the (3) anionic substance has the function of allowing migration and orientation of the cultured cells and of suppressing cytotoxicity.

According to this mixed-type temperature-responsive polymer composition, the cloud point can be reduced to room temperature (25°C) or below.

In the aforementioned composition, it is inferred that the side chain of the polymer of DMAEMA and/or a derivative thereof and the tris interact with each other (for example, by crosslinking), making it easier for the polymer to aggregate.

In (1), the polymer of DMAEMA and/or a derivative thereof is preferably a molecule with a number-average molecular weight (Mn) of 10 kDa to 500 kDa. The polymer of DMAEMA and/or a derivative thereof is also preferably a molecule for which the ratio (Mw/Mn) of the weight-average molecular weight (Mw) to the number-average molecular weight (Mn) is 1.1 to 6.0.

Examples of the (1) derivative of DMAEMA include a derivative in which a hydrogen atom of the methyl group of methacrylate is replaced by a halogen atom, a derivative in which the methyl group of methacrylate is replaced by a lower alkyl group, a derivative in which the hydrogen atom of the methyl group of a dimethylamino group is replaced by a halogen atom, and a derivative in which the methyl group of a dimethylamino group is replaced by a lower alkyl group.

The (2) tris is preferably is a pure substance with a 99.9% or higher purity or is a tris aqueous solution that is made neutral or basic at the time of use, for example by addition of an alkaline substance. When using tris in its commercially available state of hydrochloride, the pH of the tris aqueous solution lowers, and the cloud point of the composition ends up rising to approximately 70°C. Therefore, a tris aqueous solution is not preferred.

Among the examples of anionic substances listed above in (3), examples of the nucleic acids include DNA, RNA, and artificial nucleic acids such as single-stranded, double-stranded, oligomer, and hairpin nucleic acids.

In particular, the DNA preferably differentiates stem cells, such as iPS cells, ES cells, and mesenchymal stem cells, and is particularly preferably DNA capable of inducing differentiation into cardiomyocytes, hepatocytes, nerve cells, and vascular endothelial cells.

The anionic substances listed above in (3) preferably have a certain size, such as a molecular weight (M) of 1 kDa to 5,000 kDa.

Setting the molecular weight in this range allows the anionic substance to undergo ionic bonding with the cationic substance and fulfill the role of trapping the cationic substance for an extended period of time. Stable microparticles of an ion complex can thus be formed. The cytotoxicity of a typical cationic substance, caused by electrostatic interaction with the cell surface membrane of a cell, can also be mitigated.

In addition to the anionic substances listed in (3), it is also possible to use a polymer derivative, for example, that substantially functions as an anionic substance by introducing an anionic functional group into an amino group in the 4-position of poly(4-aminostyrene), which is a cationic polymer, by dehydration synthesis of dicarboxylic acid of oxalic acid or the like.

Two or more types of the anionic substances listed above in (3) may be included.

Here, a mixed-type temperature-responsive polymer composition in which the ratio ((2)/(1)) of (2) 2-amino-2-hydroxymethyl-1,3-propanediol (tris) to (1) a polymer of 2-N,N-dimethylaminoethyl methacrylate (DMAEMA) and/or a derivative thereof is 1.0 or less is preferably used.

The ratio ((2)/(1)) is designated as the weight ratio.

When using a mixed-type temperature-responsive polymer composition with the above ratio, the cellular structure can be formed more easily in the below-described culturing step.

According to this composition, the balance between hydrophilicity and hydrophobicity of the composition can be further improved. It is inferred that this suitable balance suitably adjusts the adhesiveness of cells to the culture surface and activates migration and orientation of the cells.

The above ratio ((2)/(1)) is preferably 0.1 or greater.

Setting the above ratio to 0.1 or greater facilitates achievement of the aforementioned effect of reducing the cloud point and also facilitates achievement of the aforementioned effect of easier formation of cellular structures.

For the same reasons as above, the above ratio ((2)/(1)) is more preferably 0.1 to 0.5.

The C/A ratio (positive/negative charge) in the mixed-type temperature-responsive polymer composition is preferably 0.5 to 16.

In the present disclosure, the C/A ratio refers to the ratio of the positive charge of material included in the composition to the negative charge of material included in the composition. Specifically, the C/A ratio is represented by the expression ((positive charge per polymer molecule) × N1)/((negative charge per molecule of anionic substance) × N3), where N1 is the number of moles of (1) the polymer of DMAEMA and/or a derivative thereof, and N3 is the number of moles of the anionic substance.

Furthermore, when the anionic substance is DNA in the present disclosure, the number of negative charges per molecule of the anionic substance is calculated as the number of base pairs (bp number) of DNA × 2, and the molecular weight (Da) is calculated as the bp number × 660 (the average molecular weight of an AT pair and a CG pair).

Setting the C/A ratio to be 0.5 to 16 facilitates achievement of the aforementioned effect of easier formation of tubular cellular structures.

It is inferred that this range makes the balance between negative charge and positive charge in the composition suitable and can suppress cytotoxicity due to positive charge. It is also inferred that this range further improves the balance between hydrophilicity and hydrophobicity of the composition and can facilitate cell migration and orientation.

For the same reasons as above, the above ratio C/A is more preferably 2 to 10 and most preferably near 8.

Moisture may be removed from the temperature-responsive polymer or the temperature-responsive polymer composition by heating or freeze drying, vacuum distillation, or the like, and the result may be dissolved in an organic solvent, examples of which include methanol, ethanol, and other alcohols; ketone; and ester. Among these, dissolving in methanol is preferable since methanol has a low surface tension and boiling point, allows rapid drying, and can more uniformly cover the temperature-responsive polymer. When dissolving in an organic solvent, hydrophilic molecules that are non-ionic and hydrophilic, such as polyethylene glycol (PEG), dimethyl acrylamide (DMAA), glycerin, Triton X, polypropylene glycol, and the like may be further added.

### (Manufacturing method of a cell culture container)

FIG. 1 illustrates an outline of an example of a manufacturing method of a cell culture container according to an embodiment of the present disclosure, along with an outline of an example of a cell culture container according to an embodiment of the present disclosure along and an outline of an example of a cell culture method using a cell culture container according to an embodiment of the present disclosure.

The example of a manufacturing method of a cell culture container according to an embodiment of the present disclosure includes only first coated regions, without including second coated regions.

In the example of a manufacturing method of a cell culture container according to an embodiment of the present disclosure, a cell culture container is first prepared (see (i) in FIG. 1).

In the example illustrated in FIG. 1, the culture surface of the cell culture container is cell non-adhesive.

Next, the temperature-responsive polymer or temperature-responsive polymer composition is spotted onto a plurality of locations on the culture surface of the cell culture container (see (ii) in FIG. 1).

Examples of a method of spotting the temperature-responsive polymer or temperature-responsive polymer composition include a procedure with an eight-channel micropipettor, spotter printing to discharge solution quantitatively, and rotating screen drum printing.

When used in the state of an aqueous solution, the temperature-responsive polymer or temperature-responsive polymer composition may be used after being cooled to its cloud point or lower.

In the example illustrated in FIG. 1, an aqueous solution of the temperature-responsive polymer is applied at four locations.

As described above, examples of the coating method include precipitation from the state of an aqueous solution, removal (drying) of the solvent from an applied aqueous solution or organic solvent solution, exposure to radiation, exposure to low temperature plasma, corona discharge, glow discharge, ultraviolet light, or graft polymerization using a radical generating agent.

In an example of this manufacturing method, the temperature-responsive polymer is bonded to the culture surface of the cell culture container (by interaction, non-covalent bonds, covalent bonds, or the like) (see (iii) of FIG. 1).

In the example illustrated in FIG. 1, an aqueous solution of the applied temperature-responsive polymer is dried.

The application amount may be 0.005 µL to 200 µL and is preferably 1 µL to 50 µL.

FIG. 1 illustrates an outline of an example of a cell culture method using a cell culture container according to an embodiment of the present disclosure, along with an outline of a cell culture container according to an embodiment of the present disclosure and an outline of an example of a manufacturing method of a cell culture container according to an embodiment of the present disclosure.

In the example of a cell culture method using a cell culture container according to an embodiment of the present disclosure, cells are seeded (see (iv) in FIG. 1). Here, a medium may be added to the cell culture container, and a medium in which cells are suspended may be added subsequently.

The density of seeded cells is preferably 1,500 cells/mm² or less (3.0 × 10⁵ cells/mL or less in the case of seeding by adding a 1.0 mL cell suspension to a 24-well cell culture plate with a culture surface area of 200 mm²). Live cells are seeded.

Adopting the aforementioned cell density facilitates formation of a cellular structure with an aggregated (pellet-like) structure in the cells that adhere to the first coated regions.

This example of a cell culture method is particularly suitable for vascular endothelial cells, adipocytes, adipose stem cells, fibroblasts, and other mesenchymal cells. In the case of primary cells, it suffices to select adherent cells that form colonies, which a person skilled in the art can appropriately select.

Subsequently, in this example of a cell culture method, the seeded cells are cultured (see (v) in FIG. 1). The culture conditions may be determined appropriately in accordance with the cells being used. An example is 37°C and a 5% CO₂ atmosphere.

At this time, as illustrated in (v) of FIG. 1, the seeded cells that are positioned in the first coated regions within the culture surface adhere to the first coated regions, whereas the seeded cells that are positioned on the non-coated region, which is cell non-adhesive, within the culture surface do not adhere to the culture surface.

Here, in this example of a cell culture method, the medium is replaced (see (vi) of FIG. 1).

At this time, as illustrated in (vi) of FIG. 1, the cells that did not adhere to the culture surface are removed from the cell culture container.

In this example of a cell culture method, the cells that adhered to the first coated regions are further cultured (see (vii) of FIG. 1). The culture conditions may be determined appropriately in accordance with the cells being used. An example is 37°C and a 5% CO₂ atmosphere.

At this time, cellular structures that have an aggregated (pellet-like) structure can easily be formed in the first coated regions that have a surface zeta potential within a particular range.

The cellular structures formed in this way subsequently detach spontaneously from the first coated regions and move along the culture surface of the cell culture container (see (viii) in FIG. 1).

Furthermore, a plurality of cellular structures moving along the culture surface come into contact and adhere to each other, forming a larger cellular structure (see (ix) in FIG. 1). Here, contact between a plurality of cellular structures may be encouraged by appropriately tipping and mixing the cell culture container.

FIG. 8 illustrates an outline of another example of a manufacturing method of a cell culture container according to an embodiment of the present disclosure, along with an outline of another example of a cell culture container according to an embodiment of the present disclosure and an outline of another example of a cell culture method using a cell culture container according to an embodiment of the present disclosure.

Another example of a cell culture container according to an embodiment of the present disclosure includes second coated regions inside the first coated regions.

In another example of a manufacturing method of a cell culture container according to an embodiment of the present disclosure, the preparation of the cell culture container (see (i) in FIG. 8), spotting of the temperature-responsive polymer or temperature-responsive polymer composition on the culture surface of the cell culture container (see (ii) in FIG. 8), and joining of the temperature-responsive polymer to the culture surface of the cell culture container (see (iii) in FIG. 8) may be similar to the above-described example of a manufacturing method of a cell culture container according to an embodiment of the present disclosure.

Subsequently, in this other example of a manufacturing method, cell adhesive material is spotted onto the first coated regions (see (iv) in FIG. 8).

Examples of a method of spotting the temperature-responsive polymer or temperature-responsive polymer composition include a procedure with an eight-channel micropipettor, spotter printing to discharge solution quantitatively, and rotating screen drum printing.

In the example illustrated in FIG. 8, an aqueous solution of cell adhesive material is applied to one location in each of four first coated regions.

The application amount may be 0.01 µL to 100 µL and is preferably 0.1 µL to 10 µL.

Subsequently, in this other example of a manufacturing method, the applied aqueous solution of cell adhesive material is dried, as in the example illustrated in FIG. 1.

FIG. 8 illustrates an outline of another example of a cell culture method using a cell culture container according to an embodiment of the present disclosure, along with an outline of another example of a cell culture container according to an embodiment of the present disclosure and an outline of another example of a manufacturing method of a cell culture container according to an embodiment of the present disclosure.

In another example of a manufacturing method of a cell culture container according to an embodiment of the present disclosure, the seeding of cells (see (vi) in FIG. 8), culturing of seeded cells (see (vii) in FIG. 8), medium replacement (see (viii) in FIG. 8), and further culturing of cells adhered to the first and second coated regions (see (ix) in FIG. 8) may be similar to the above-described example of a manufacturing method of a cell culture container according to an embodiment of the present disclosure.

As described above, among the formed cellular structures, a portion of the cellular structures positioned in the second coated region are anchored to the cell culture container.

A cell culture container, manufactured by an example of a manufacturing method of a cell culture container according to an embodiment of the present disclosure, that includes only the first coated regions and does not include the second coated regions can be used suitably in the fields of regenerative medicine and drug delivery (DDS).

Specifically, the cell culture container could be used as the cell source for a site to be regenerated by regenerative medicine or used to secrete cytokines in DDS.

A cell culture container, manufactured by another example of a manufacturing method of a cell culture container according to an embodiment of the present disclosure, that includes the second coated regions within the first coated regions can be used suitably in the field of drug discovery.

Specifically, the cell culture container could be used for small-scale drug screening using high-cost iPS cells.

FIGS. 12A and 12B illustrate an outline of a cellular structure culture vessel according to an embodiment of the present disclosure using another example of a cell culture container according to an embodiment of the present disclosure. FIG. 12A is a perspective view of the cellular structure culture vessel, and FIG. 12B is a cross-sectional view of the cellular structure culture vessel.

As illustrated in FIGS. 12A and 12B, the cellular structure culture vessel of an embodiment of the present disclosure includes a stack of a plurality of layers of culture sheets having a plurality of locations with a second coated region inside a first coated region.

The cellular structure culture vessel of an embodiment of the present disclosure can suitably be used to culture cellular structures in a high state of activity, to cause the cellular structures to generate a desired substance or biological material (for example, growth factors, peptides, sugars, proteins, cytokines, other physiologically active substances, exosomes, and microRNAs), and to obtain the substance or biological material.

In a method using a conventional culture vessel, a medium including a cell mass is stirred within a container to float and disperse the cell mass, obtaining an anticoagulant (t-PA or the like) or other substance produced by the cell mass. However, since adherent cells are also caused to float without adhering, the cell activity reduces, resulting in a low production efficiency of the desired substance. One improved method that has been studied to resolve this issue is to adhere cells to beads and to disperse the cells while stirring a medium that includes the beads. This method, however, also has a relatively large culture scale and has a high risk of the cells being infected by bacteria. It is also difficult to adhere the cells efficiently and uniformly to the beads.

According to the cellular structure culture vessel of an embodiment of the present disclosure, adherent cells can be cultured after adhering to the cell culture surface, and since the medium does not need to be stirred, the risk of cells being infected by bacteria can be reduced while effectively using the space within the container.

Details on the culture sheets in the cellular structure culture vessel may be as in the above-described other example of a cell culture container according to an embodiment of the present disclosure.

The aforementioned locations at which the cellular structure is anchored in the culture sheets are preferably arrayed in the horizontal and vertical directions of the sheets for a uniform culture environment.

In the cellular structure culture vessel, a space large enough for the medium to pass through is provided between the culture sheets. This space may, for example, be provided by appropriately forming projections with a greater height than the diameter of the cellular structures on the front and/or back of the culture sheet and having the tip of the projections on one culture sheet support the adjacent culture sheet.

As illustrated in FIGS. 12A and 12B, an injection port for injecting a medium and an ejection port for ejecting the medium are provided on the side walls of the container in the cellular structure culture vessel.

As illustrated in FIGS. 12A and 12B, in an example of culturing cellular structures using the cellular structure culture vessel of an embodiment of the present disclosure, a medium is injected through the injection port and ejected through the ejection port after preparing cellular structures anchored to the culture sheets.

A desired substance or biological material may be extracted by subjecting the ejected medium to a method known to a person skilled in the art, such as moisture evaporation and concentration, freeze drying, ethanol precipitation, phenol treatment, antibody-fixed magnetic bead treatment, antibody-fixed fluorescent bead treatment, gel filtration, centrifugation, a densigy gradient, dialysis, filtration, electrophoresis, enzyme treatment, and liquid-liquid extraction.

The ejected medium may be perfused, or optionally may be reused after being dialyzed to remove ammonia and other metabolites and/or after bubbling oxygen to increase the dissolved oxygen concentration. A new medium may also be injected.

The cellular structure culture vessel of an embodiment of the present disclosure can suitably be used for production of biologically derived physiologically active substances or other such uses.

### EXAMPLES

The present disclosure is described in more detail below with reference to Examples, by which the present disclosure is not intended to be limited in any way.

In the following tests, commercially available reagents were used without further purification, unless otherwise noted.

### (Example 1)

### (Test 1-1) Polymer manufacturing-1

### Manufacturing of intramolecular ion complex-type temperature-responsive polymer (Example manufacturing process 1-1)

First, 10.0 g of 2-N,N-dimethylaminoethyl methacrylate (DMAEMA) was added to a 50 mL capacity transparent vial made of soft glass, and the vial was stirred using a magnetic stirrer.

The mixture (liquid) was then purged with G1-grade, highly pure (purity: 99.99995%) nitrogen gas for 10 minutes (flow rate: 2.0 L/min) to remove oxygen from the mixture.

The aforementioned reactant was then polymerized by irradiating the mixture with ultraviolet light for five hours using a round black fluorescent lamp (model FCL20BL by NEC Corporation, 18 W).

Here, a portion of the reactant was collected and the number-average molecular weight (Mn) of the polymerized DMAEMA was measured as 970,000 (dispersion = 4.1).

Immediately after confirming that the number-average molecular weight has reached at least 5,000, 1.4 g of methacrylic acid was added while maintaining the inside of the vial under an inert atmosphere by causing an inert gas to flow into the vial. The vial was then stirred again using a magnetic stirrer. The methacrylic acid added at this point had been subjected to bubbling with an inert gas.

The aforementioned reactant was polymerized by irradiating the mixture with ultraviolet light for 16 hours using a round black fluorescent lamp (model FCL20BL by NEC Corporation, 18 W). The reactant became viscous immediately after mixing and hardened after one hour. A polymer was thus obtained as a reaction product. This reaction product was dissolved in 2-propanol, and the solution was transferred to a dialysis tube. Dialysis was performed for 72 hours to purify the reaction product.

The solution including the reaction product was filtered with a 0.2 µm cellulose mixed-ester filter (model 25AS020 by Toyo Roshi Kaisha, Ltd.), and the resulting filtrate was freeze dried to obtain a temperature-responsive polymer composed of homopolymer blocks of DMAEMA and copolymer blocks of DMAEMA and methacrylic acid (6.8 g yield, 60% conversion ratio).

The number-average molecular weight (Mn) of this polymer was measured using a GPC (model LC-10vp series by Shimadzu Corporation) with polyethylene glycol (TSK series by Shodex) as a standard substance and was determined to be Mn = 4,500,000 (Mw/Mn = 2.2) (Example polymer 1-1).

The nuclear magnetic resonance (NMR) spectrum of Example polymer 1-1 was measured using a nuclear magnetic resonance apparatus (model Gemini-300 by Varian) with heavy water (D₂O) as a standard substance. The representative peaks common to Example polymer 1-1 are listed below.
¹H-NMR (in D₂O) δ 0.8-1.2 (br, 3H, -CH₂-C(CH₃)-), 1.6-2.0 (br, 2H, -CH₂-C(CH₃)-), 2.2-2.4 (br, 6H, -N(CH₃)₂), 2.5-2.7 (br, 1.9H, -CH₂-N(CH₃)₂), 4.0-4.2 (br, 1.9H, -O-CH₂-).

Here, from the number of protons A in the methyl group (δ 0.8-1.2) bonded at the α position (three in both the case of a DMAEMA unit and the case of a methacrylic acid unit) and the number of methyl protons B in the ethyl group (δ 4.0-4.2) bonded to oxygen in the ester bond of a side chain (two in the case of a DMAEMA unit and zero in the case of a methacrylic acid unit), the ratio between the number of functional groups that are amino groups in the side chains of DMAEMA and the number of functional groups that are carboxyl groups in the side chains of methacrylic acid was calculated.

The result was a ratio of 94:6 for the Example polymer 1-1. Converting into the C/A ratio for an ion complex unit in a two-component mixed system that includes a cationic polymer and an anionic polymer yields a C/A ratio of 15.6.

### (Test 2) Measurement of cloud point of polymer

A 3% aqueous solution of Example polymer 1-1 was prepared, and the absorbance of the aqueous solution at 660 nm was measured between 20°C and 40°C.

Between 20°C and 30°C, the aqueous solution was transparent, with an absorbance of nearly 0. Starting around 31°C, however, the aqueous solution became cloudy, and the absorbency increased suddenly at 32°C. The aforementioned polymer was thus confirmed to have a cloud point of approximately 32°C.

Once the Example polymer was increased in temperature to 37°C, the polymer aqueous solution was suspended with good responsiveness. Subsequently, the entire aqueous solution hardened. When maintained at room temperature (25°C), the hardened product retained its hard state for several tens of hours. Subsequently, the hardened product gradually dissolved, changing into a uniform aqueous solution. Upon being cooled to 4°C, the hardened polymer rapidly dissolved. Repeating the aforementioned operation to raise and lower the temperature caused no change in responsiveness, thereby confirming that the polymer reversibly underwent phase transitions.

### (Test 3) Particle size measurement of polymer aggregates

The particle size of aggregates of polymer molecules was 250 nm as measured by light scattering using a static light scattering apparatus (model Zetasizer nano by Sysmex Corporation) with the Example polymer 1-1 as a 20°C liquid. This suggests that even at a temperature of 20°C, which is below the cloud point, aggregates with a relatively large particle size are formed, i.e. aggregates that tend to precipitate and that tend not to diffuse after precipitation. This also suggests that the Example polymer 1 can easily coat a cell culture container.

### (Test 4) Manufacturing of a cell culture container (cell culture container having only first coated regions)

A 35 mm polystyrene cell culture plate (model 3000-035-MYP by Iwaki & Co., Ltd, bottom area of 9 cm² per well) was used as a cell culture container.

The culture surface was coated with a non-ionic surfactant (Pluronic F-68 by Asahi Denka Corporation) so that the culture surface was cell non-adhesive.

Next, a micropipettor was used to apply 4 µL of aqueous solution (concentration: 15 µg/mL) of temperature-responsive polymer, cooled to the cloud point or below, in circles at 28 locations on the culture surface.

The radius of the circular first coated region at each location was approximately 2,000 µm, and the distance between first coated regions was approximately 1,000 µm.

The applied aqueous solution of temperature-responsive polymer was dried by leaving the cell culture plate on a clean bench.

First coated regions that were coated with the temperature-responsive polymer were thus provided on the culture surface of the cell culture plate.

FIG. 2 is a view from the culture surface of the cell culture container of an example of the present disclosure produced in Test 4.

### (Test 5) Measurement of zeta potential

The surface zeta potential of first coated regions, which were provided on a small piece of a cell culture plate in accordance with the same procedure as the procedure for Test 4, was measured using a zeta potential meter (model ELSZ by Otsuka Electronics Co., Ltd) and a cell unit for flat plate samples.

Specifically, a sample of the small piece was tightly adhered to the bottom surface of a quartz cell, and a monitor particle suspension was injected into the cell. Here, particles (zeta potential: -5 mV to +5 mV) yielded by coating polystyrene latex (particle size: approximately 500 nm) with hydroxypropyl cellulose (Mw = 30,000) were used as standard monitoring particles. As a solvent, a 10 mM aqueous solution of sodium chloride was used under the conditions of pH = 7, 37°C. The zeta potential was calculated using the Smoluchowski formula.

The zeta potential of the surface of a small piece of non-coated cell culture plate is -68 mV, which is a value known to a person skilled in the art as the zeta potential of a solid surface of a typical thermoplastic resin.

In contrast, the zeta potential of the surface of the small piece of cell culture plate coated with a temperature-responsive polymer was +20 mV.

As a person skilled in the art knows, the measured value of the zeta potential of a solid surface exhibits approximately ±10% variation with current techniques. Variation is also present in the coating operation itself during the step of preparing the sample. Hence, the aforementioned measurement of the zeta potential may have a certain error.

### (Test 6) Measurement of contact angle

The contact angle of water relative to the first coated region of a cell culture plate was measured as 70° ± 10° using a contact angle meter (product name DMs-400, by Kyowa Interface Science Co., Ltd.) in conformity with JIS R3257.

### (Test 7) Cell culture

Here, 2.5 × 10⁶ mesenchymal adipose stem cells, derived from rat subcutaneous fat and tagged with GFP, were suspended in a complete medium (a liquid of Dulbecco's Modified Eagle Medium (DMEM) + 10% fetal bovine serum (FBS); DMEM: model 11995-065 by Gibco; FCS: lot number 928696, by Invitrogen) and were seeded to achieve a cell density of 2.5 × 10⁶ cells/plate.

The seeded adipose stem cells derived from rat subcutaneous fat were cultured for three hours in a cell culture incubator at 37°C in a 5% CO₂ atmosphere.

At this time, the seeded cells that were positioned in the first coated regions within the culture surface adhered to the first coated regions, whereas the seeded cells that were positioned on the non-coated region, which is cell non-adhesive, within the culture surface did not adhere to the culture surface.

After culturing for three hours, the medium was replaced using a new DMEM + 10% FCS liquid.

FIGS. 3A to 3D and FIGS. 4A to 4D are photographs taken using a phase contrast microscope to observe the state when culturing adipose stem cells derived from rat subcutaneous fat in first coated regions of a cell culture container of an example of the present disclosure.

FIGS. 3A to 3D and FIGS. 4A to 4D respectively illustrate the state at zero hours (immediately after medium replacement), 2 hours, 3 hours, 4 hours, 5 hours, 6 hours, 8 hours, and 12 hours after medium replacement.

At zero hours after medium replacement (immediately after medium replacement), the adipose stem cells were adhered to the entire surface of the first coated regions (see FIG. 3A).

At 2 hours after medium replacement, in particular the cells, among the adhered cells, that were positioned at the outer edge of the first coated regions spontaneously started to detach (see FIG. 3B).

From 3 hours to 6 hours after medium replacement, spontaneous detachment of cells progressed slowly from the outer edge of the first coated regions towards the center of the first coated regions (see FIGS. 3C, 3D, 4A, and 4B).

At 8 hours after medium replacement, cellular structures having an aggregated (pellet-like) structure ended up forming in the cultures in the first coated regions, the cellular structures being equal in number to the number of first coated regions (see FIG. 4C).

At 12 hours after medium replacement, the aggregated cellular structures spontaneously started to detach from the first coated regions and move on the culture surface of the cell culture container. Some of the cellular structures started to come into contact with each other (see FIG. 4D).

FIG. 7A is a photograph taken using a stereomicroscope to observe the state zero hours after medium replacement (immediately after medium replacement) when culturing adipose stem cells derived from rat subcutaneous fat in the first coated regions of a cell culture container of an example of the present disclosure.

In this example, the aggregated cellular structures were further cultured in a cell culture incubator at 37°C in a 5% CO₂ atmosphere.

FIGS. 5A to 5D and 6A to 6D are photographs taken using a phase contrast microscope to observe the state when culturing adipose stem cells derived from rat subcutaneous fat in first coated regions of a cell culture container of an example of the present disclosure.

FIGS. 5A to 5D and FIGS. 6A to 6D respectively illustrate the state at zero hours, 5 hours, 8 hours, 12 hours, 17 hours, 22 hours, 25 hours, and 34 hours after aggregated cellular structures detach from the first coated regions spontaneously and float, and the cell culture container is appropriately tipped and mixed so that the suspended matter is artificially collected in the center of the cell culture container and the cellular structures come into contact with each other.

When multiple aggregated cellular structures moved on the culture surface and came into contact, they adhered to each other and formed a larger cellular structure. The cellular structures repeated this phenomenon until forming several aggregated cellular structures (see FIGS. 5A to 5D and FIGS. 6A to 6D).

FIG. 7B is a photograph taken using a stereomicroscope to observe the state 21 hours after medium replacement when culturing adipose stem cells derived from rat subcutaneous fat in the first coated regions of a cell culture container of an example of the present disclosure.

### (Test 8) Manufacturing of a cell culture container (cell culture container having second coated regions in the first coated regions)

As in the above-described Test 4, first coated regions that were coated with the temperature-responsive polymer were provided on the culture surface of a cell culture plate.

Next, an ultra-trace amount pipettor (Digifit by NICHIRYO) was used to apply 0.2 µL of an aqueous solution of fibronectin derived from human plasma (by BD Falcon, concentration: 1 mg/mL, lot number: 3353663) at a time in circles, inside the respective circular first coated regions provided at 28 locations on the culture surface. The aqueous solution was applied so that the circles of the first coated regions and the circles of the second coated regions were concentric.

The diameter of one circular second coated region was approximately 250 µm.

The applied aqueous solution of cell adhesive material was dried by leaving the cell culture plate on a clean bench.

Second coated regions were thus provided within the first coated regions that were coated with the temperature-responsive polymer on the culture surface of the cell culture plate.

### (Test 9) Cell culture

Here, as in the above-described Test 4, 2.5 × 10⁶ adipose stem cells, derived from rat subcutaneous fat and tagged with GFP, were suspended in a complete medium (a liquid of Dulbecco's Modified Eagle Medium (DMEM) + 10% fetal bovine serum (FBS); DMEM: model 11995-065 by Gibco; FCS: lot number 928696, by Invitrogen) and were seeded to achieve a cell density of 2.5 × 10⁶ cells/plate.

The seeded adipose stem cells derived from rat subcutaneous fat were cultured for three hours in a cell culture incubator at 37°C in a 5% CO₂ atmosphere.

At this time, the seeded cells that were positioned in the first coated regions within the culture surface adhered to the first coated regions, whereas the seeded cells that were positioned on the non-coated region, which is cell non-adhesive, within the culture surface did not adhere to the culture surface.

After culturing for three hours, the medium was replaced using a new DMEM + 10% FCS liquid.

Photographs (not illustrated) taken using a phase contrast microscope to observe the state when culturing adipose stem cells derived from rat subcutaneous fat in the first coated regions and second coated regions of a cell culture container of an example of the present disclosure were nearly identical to those of FIGS. 3A to 3D and FIGS. 4A to 4D.

The state (not illustrated) at zero hours (immediately after medium replacement), 2 hours, 3 hours, 4 hours, 5 hours, 6 hours, 8 hours, and 12 hours after medium replacement was nearly identical to that of FIGS. 3A to 3D and FIGS. 4A to 4D.

At zero hours after medium replacement (immediately after medium replacement), the adipose stem cells were adhered to the entire surface of the first coated regions (see FIG. 3A).

At 2 hours after medium replacement, in particular the cells, among the adhered cells, that were positioned at the outer edge of the first coated regions spontaneously started to detach (see FIG. 3B).

From 3 hours to 6 hours after medium replacement, spontaneous detachment of cells progressed slowly from the outer edge of the first coated regions towards the center of the first coated regions (see FIGS. 3C, 3D, 4A, and 4B).

At 8 hours after medium replacement, cellular structures having an aggregated (pellet-like) structure ended up forming in the cultures in the first coated regions, the cellular structures being equal in number to the number of first coated regions (see FIG. 4C).

At 12 hours after medium replacement, the majority of the aggregated cellular structures had detached spontaneously from the first coated regions, but the portion of the cellular structures positioned on the second coated regions was anchored to the second coated regions and remained above the first and second coated regions (see FIG. 9).

FIG. 9 is a photograph taken using a stereomicroscope to observe the state zero hours after medium replacement (immediately after medium replacement) when culturing adipose stem cells derived from rat subcutaneous fat in the first and second coated regions of a cell culture container of an example of the present disclosure.

In this example, the aggregated cellular structures were cultured for a predetermined length of time in a cell culture incubator at 37°C in a 5% CO₂ atmosphere.

Even when appropriately tipping and mixing the cell culture container, the formed cellular structures did not detach from the cell culture dish, and the cellular structures that were formed in adjacent first and second coated regions did not adhere.

FIGS. 10A to 10D are photographs taken using a phase contrast microscope to observe the state when the cellular structure illustrated in FIG. 9 was shaken by hand in all four directions in a horizontal plane at a speed of approximately 10 cm/s, respectively illustrating the state at 0 s, 0.5 s, 1.0 s, and 1.5 s after the start of shaking.

FIGS. 11A to 11D are photographs taken using a phase contrast microscope to observe the state when the cellular structure illustrated in FIG. 9 was shaken by hand in all four directions in a horizontal plane at a speed of approximately 10.0 cm/s, respectively illustrating the state at 2.0 s, 2.5 s, 3.0 s, and 3.5 s after the start of shaking.

As is clear from FIGS. 10A to 10D and FIGS. 11A to 11D, during shaking, six aggregated cellular structures (see the top of the culture surface in the figures) were not displaced relative to the cell culture dish, whereas cells other than the cellular structures on the cell culture dish (see the bottom right of the culture surface in the figures) were displaced relative to the cell culture dish.

### (Example 2 to Example 11)

Conditions were changed as listed in Table 1 below, and the same operations as those listed for Test 1 to Test 9 were performed, as in Example 1.

Details on the experiment materials used in Example 2 to Example 11 are listed below.

### -Cell culture container-

- Low-adhesion plate (35 mm): Prime Surface® (Prime Surface is a registered trademark in Japan, other countries, or both) (Sumitomo Bakelite Co., Ltd.)
- Glass Petri dish (50 mm): BROSIL (AS ONE Corporation)
- General-purpose sterilized (for Escherichia coli) Petri dish (90 mm): GD90-15 (AS ONE Corporation)

### -Temperature-responsive polymer or temperature-responsive polymer composition-

### • Example polymer 1-2: prepared with Test 1-2 below.

### (Test 1-2) Polymer manufacturing-2

First, 10.0 g of 2-N,N-dimethylaminoethyl methacrylate (DMAEMA) and 5,000 µL of water were added to a 50 mL capacity transparent vial made of soft glass, and the vial was stirred using a magnetic stirrer. The mixture (liquid) was then purged with G1-grade, highly pure (purity: 99.99995%) nitrogen gas for 10 minutes (flow rate: 2.0 L/min) to remove oxygen from the mixture. The DMAEMA that was used included 0.5% by weight of methylhydroquinone (MEHQ), which is a polymerization inhibitor.

Subsequently, this reactant was polymerized by irradiating the reactant with ultraviolet light for 22 hours using a round black fluorescent lamp (model FCL20BL by NEC Corporation, 18 W). The reactant became viscous 5 hours after mixing and hardened after 15 hours. A polymer was thus obtained as a reaction product. This reaction product was dissolved in 2-propanol, and the solution was transferred to a dialysis tube. Dialysis was performed for 72 hours to purify the reaction product.

The solution including the reaction product was filtered with a 0.2 µm cellulose mixed-ester filter (model 25AS020 by Toyo Roshi Kaisha, Ltd.), and the resulting filtrate was freeze dried to obtain an intramolecular ion complex-type temperature-responsive polymer (6.8 g yield, 68% conversion ratio). The number-average molecular weight (Mn) of this polymer was measured using a GPC (model LC-10vp series by Shimadzu Corporation) with polyethylene glycol (TSK series by Shodex) as a standard substance and was determined to be Mn = 100,000 (Mw/Mn = 10.0).

The cloud point and particle size of the polymer were similar to the case of Example polymer 1-1.
- Example polymer 1-3: The purge time of the nitrogen gas in Test 1-2 was changed from 10 minutes to 20 minutes. Hydrolysis progressed more than polymerization, and the anionic component increased.
- Example polymer 1-4: The purge time of the nitrogen gas in Test 1-2 was changed from 10 minutes to 5 minutes. Polymerization progressed more than hydrolysis, and the cationic component increased.
- Example polymer 1-5: Instead of the DMAEMA in Test 1-2, DMAEMA having compounded therein 3% by mass of N,N-dimethyl acrylamide (DMAA), which is a hydrophilic non-ionic monomer, was used.
- Example polymer 1-6: Instead of the DMAEMA in Test 1-2, DMAEMA having compounded therein 5% of N-isopropyl acrylamide (NIPAM), which is a non-ionic monomer, was used.

### -Cells-

- Human cancer-derived cell line (HepG2): HEPG2-500 by CET
- Cardiac muscle cells: primary cells derived from a beagle
- Chondrocytes: primary cells derived from a beagle

### -Cell adhesive material-

- Laminin: Nippi Inc.
- Collagen: Nitta Gelatin Inc.

**[Table 1-1]**

| | | | | Example 1 | Example 2 | Example 3 | Example 4 | Example 5 | Example 6 |
|---|---|---|---|---|---|---|---|---|---|
| Culture container | Cell culture container serving as a base | | - | polystyrene cell culture plate (35 mm) | low-adhesion plate (35 mm) | low-adhesion plate (35 mm) | low-adhesion plate (35 mm) | low-adhesion plate (35 mm) | glass Petri dish (50 mm) |
| | Name of above-listed product (manufacturer) | | - | 3000-035-MYP (Iwaki & Co., Ltd.) | Prime Surface® (Sumitomo Bakelite Co., Ltd.) | Prime Surface® (Sumitomo Bakelite Co., Ltd.) | Prime Surface® (Sumitomo Bakelite Co., Ltd.) | Prime Surface® (Sumitomo Bakelite Co., Ltd.) | BROSIL (AS ONE Corporation) |
| | Coating material | | - | Pluronic F-68 | none (unnecessary) | none (unnecessary) | none (unnecessary) | none (unnecessary) | none (unnecessary) |
| | First coated regions | Polymer or polymer composition | - | Example polymer 1-1 ((A-2) in description) | Example polymer 1-2 ((A-1) in description) | Example polymer 1-2 ((A-1) in description) | Example polymer 1-2 ((A-1) in description) | Example polymer 1-2 ((A-1) in description) | Example polymer 1-2 ((A-1) in description) |
| | | Surface zeta potential | mV | +20 | +20 | +20 | +20 | +20 | +20 |
| | | Contact angle of water | ○ | 70±10 | 70±10 | 70±10 | 70±10 | 70±10 | 70±10 |
| | | Area | mm² | 3.14 | 3.14 | 3.14 | 3.14 | 3.14 | 3.14 |
| | | Number (of locations) | number | 28 | 28 | 28 | 28 | 28 | 28 |
| | | Shape | - | circle (diameter: 2000 µm) | circle (diameter: 2000 µm) | circle (diameter: 2000 µm) | circle (diameter: 2000 µm) | circle (diameter: 2000 µm) | circle (diameter: 2000 µm) |
| | | Distance from adjacent region | µm | 1000 | 1000 | 1000 | 1000 | 1000 | 1000 |
| | | Amount of polymer per location | ng | 60 | 60 | 60 | 60 | 60 | 60 |
| Culture method | Number of seeded cells (one plate) | | number | 2.5 × 10⁶ | 2.5 × 10⁶ | 2.5 × 10⁶ | 2.5 × 10⁶ | 2.5 × 10⁶ | 4.5 × 10⁶ |
| | Type of cells | | - | GFP knock-in mesenchymal stem cells derived from rat subcutaneous fat | HepG2 | cardiac muscle cells | chondrocytes | GFP knock-in mesenchymal stem cells derived from rat subcutaneous fat | GFP knock-in mesenchymal stem cells derived from rat subcutaneous fat |
| Test 4 | Formation of aggregate cellular structures | | - | formed | formed | formed | formed (circular) | formed | formed |
| Culture container | Second coated regions | Cell adhesive material | - | fibronectin derived from human plasma | Example polymer 1-2 ((A-1) in description) | laminin | collagen | fibronectin derived from human plasma | fibronectin derived from human plasma |
| | | Area | mm² | 0.05 | 0.05 | 0.05 | 0.05 | 0.05 | 0.05 |
| | | Amount of cell adhesive material per location | ng | 200 | 50 | 300 | 300 | 200 | 200 |
| | | Number (of locations) | number | one each (total 28) | one each (total 28) | one each (total 28) | one each (total 28) | one each (total 28) | one each (total 28) |
| | | Shape | - | circle (diameter: 250 µm) | circle (diameter: 250 µm) | circle (diameter: 250 µm) | circle (diameter: 250 µm) | circle (diameter: 250 µm) | circle (diameter: 250 µm) |
| Culture method | Number of seeded cells (one plate) | | number | 2.5 × 10⁶ | 2.5 × 10⁶ | 2.5 × 10⁶ | 2.5 × 10⁶ | 2.5 × 10⁶ | 4.5 × 10⁶ |
| | Type of cells | | - | GFP knock-in mesenchymal stem cells derived from rat subcutaneous fat | HepG2 | cardiac muscle cells | chondrocytes | GFP knock-in mesenchymal stem cells derived from rat subcutaneous fat | GFP knock-in mesenchymal stem cells derived from rat subcutaneous fat |
| Test 8 | Formation of aggregate cellular structures/anchoring to culture surface | | - | formed/anchored | formed/anchored | formed/anchored | formed/anchored | formed/anchored | formed/anchored |

**[Table 1-2]**

| | | | | Example 7 | Example 8 | Example 9 | Example 10 | Example 11 |
|---|---|---|---|---|---|---|---|---|
| Culture container | Cell culture container serving as a base | | - | general-purpose sterilized (for Escherichia coli) Petri dish (90 mm) | polystyrene cell culture plate (35 mm) | polytyrene cell culture plate (35 mm) | polystyrene cell culture plate (35 mm) | polystyrene cell culture plate (35 mm) |
| | Name of above-listed product (manufacturer) | | - | GD90-15 (AS ONE Corporation) | 3000-035-MYP (Iwaki & Co., Ltd.) | 3000-035-MYP (Iwaki & Co., Ltd.) | 3000-035-MYP (Iwaki & Co., Ltd.) | 3000-035-MYP (Iwaki & Co., Ltd.) |
| | Coating material | | - | none (unnecessary) | Pluronic F-68 | Pluronic F-68 | Pluronic F-68 | Pluronic F-68 |
| | First coated regions | Polymer or polymer composition | - | Example polymer 1-2 ((A-1) in description) | Example polymer 1-3 ((A-1) in description) | Example polymer 1-4 ((A-1) in description) | Example polymer 1-5 ((A-1) in description) | Example polymer 1-6 ((A-1) in description) |
| | | Surface zeta potential | mV | +20 | +15 | +35 | +20 | +25 |
| | | Contact angle of water | ○ | 70±10 | 70±10 | 70±10 | 65±7 | 75±15 |
| | | Area | mm² | 3.14 | 3.14 | 3.14 | 3.14 | 3.14 |
| | | Number (of locations) | number | 28 | 28 | 28 | 28 | 28 |
| | | Shape | - | circle (diameter: 2000 µm) | circle (diameter: 2000 µm) | circle (diameter: 2000 µm) | circle (diameter: 2000 µm) | circle (diameter: 2000 µm) |
| | | Distance from adjacent region | µm | 1000 | 1000 | 1000 | 1000 | 1000 |
| | | Amount of polymer per location | ng | 60 | 60 | 60 | 60 | 60 |
| Culture method | Number of seeded cells (one plate) | | number | 8.5 × 10⁶ | 2.5 × 10⁶ | 2.5 × 10⁶ | 2.5 × 10⁶ | 2.5 × 10⁶ |
| | Type of cells | | - | GFP knock-in mesenchymal stem cells derived from rat subcutaneous fat | GFP knock-in mesenchymal stem cells derived from rat subcutaneous fat | GFP knock-in mesenchymal stem cells derived from rat subcutaneous fat | GFP knock-in mesenchymal stem cells derived from rat subcutaneous fat | GFP knock-in mesenchymal stem cells derived from rat subcutaneous fat |
| Test 4 | Formation of aggregate cellular structures | | - | formed | formed | formed | formed | formed |
| Culture container | Second coated regions | Cell adhesive material | - | fibronectin derived from human plasma | fibronectin derived from human plasma | fibronectin derived from human plasma | fibronectin derived from human plasma | fibronectin derived from human plasma |
| | | Area | mm² | 0.05 | 0.05 | 0.05 | 0.05 | 0.05 |
| | | Amount of cell adhesive material per location | ng | 200 | 200 | 200 | 200 | 200 |
| | | Number (of locations) | number | one each (total 28) | one each (total 28) | one each (total 28) | one each (total 28) | one each (total 28) |
| | | Shape | - | circle (diameter: 250 µm) | circle (diameter: 250 µm) | circle (diameter: 250 µm) | circle (diameter: 250 µm) | circle (diameter: 250 µm) |
| Culture method | Number of seeded cells (one plate) | | number | 8.5 × 10⁶ | 2.5 × 10⁶ | 2.5 × 10⁶ | 2.5 × 10⁶ | 2.5 × 10⁶ |
| | Type of cells | | - | GFP knock-in mesenchymal stem cells derived from rat subcutaneous fat | GFP knock-in mesenchymal stem cells derived from rat subcutaneous fat | GFP knock-in mesenchymal stem cells derived from rat subcutaneous fat | GFP knock-in mesenchymal stem cells derived from rat subcutaneous fat | GFP knock-in mesenchymal stem cells derived from rat subcutaneous fat |
| Test 8 | Formation of aggregate cellular structures/anchoring to culture surface | | - | formed/anchored | formed/anchored | formed/anchored | formed/anchored | formed/anchored |

### INDUSTRIAL APPLICABILITY

The present disclosure can provide a cell culture container capable of easily producing large quantities of minute (for example, with a size of several hundred µm or less) cellular structures (for example, spheroids).

## Claims

1. A cell culture container comprising:
a plurality of first coated regions, on a culture surface of the cell culture container, coated by a temperature-responsive polymer or a temperature-responsive polymer composition,
wherein a surface zeta potential of the first coated regions is from 0 mV to 50 mV.

2. The cell culture container of claim 1, wherein a contact angle of water relative to the first coated regions is from 50° to 90°.

3. The cell culture container of claim 1 or 2, wherein the culture surface of the cell culture container is cell non-adhesive.

4. The cell culture container of any one of claims 1 to 3, wherein a shape of the first coated regions in planar view is at least one selected from the group consisting of a circle, an ellipse, and a shape with a center of curvature on an inside of an outer contour line.

5. The cell culture container of any one of claims 1 to 4, wherein the temperature-responsive polymer or temperature-responsive polymer composition is at least one temperature-responsive polymer or temperature-responsive polymer composition selected from the group consisting of a temperature-responsive polymer containing 2-N,N-dimethylaminoethyl methacrylate (DMAEMA) units and anionic monomer units; a temperature-responsive polymer containing N-isopropyl acrylamide (NIPAM) units, cationic monomer units, and anionic monomer units; and a temperature-responsive polymer composition containing a polymer of 2-N,N-dimethylaminoethyl methacrylate (DMAEMA) and/or a derivative thereof, 2-amino-2-hydroxymethyl-1,3-propanediol (tris), and one or more anionic substances selected from the group consisting of nucleic acids, heparin, hyaluronic acid, dextran sulfate, polystyrene sulfonic acid, polyacrylic acid, polymethacrylic acid, polyphosphoric acid, sulfated polysaccharide, curdlan, polyarginic acid, and alkali metal salts thereof.

6. The cell culture container of claim 5, wherein the anionic monomer is at least one selected from the group consisting of acrylic acid, methacrylic acid, vinyl derivatives containing at least one group selected from the group consisting of a carboxyl group, a sulfonic acid group, and a phosphoric acid group in a side chain.

7. The cell culture container of claim 5 or 6, wherein the cationic monomer is at least one selected from the group consisting of 3-(N,N-dimethylaminopropyl)-(meth)acrylamide, 3-(N,N-dimethylaminopropyl)-(meth)acrylate, aminostyrene, 2-(N,N-dimethylaminoethyl)-(meth)acrylamide, and 2-(N,N-dimethylaminoethyl)-(meth)acrylate.

8. The cell culture container of any one of claims 1 to 7, further comprising second coated regions further coated with a cell adhesive material, the second coated regions being located inside the first coated regions.

9. The cell culture container of claim 8, wherein the cell adhesive material is at least one selected from the group consisting of laminin, collagen, and fibronectin.
